(19) Europäisches Patentamt
European Patent Office
Office européen des brevets

(11) **EP 4 306 144 B1**

(12) **EUROPEAN PATENT SPECIFICATION**

(45) Date of publication and mention
of the grant of the patent:
**08.04.2026 Bulletin 2026/15**

(21) Application number: **23185182.5**

(22) Date of filing: **13.07.2023**

(51) International Patent Classification (IPC):
*A61M 1/36* (2006.01)          *A61M 1/34* (2006.01)
*A61M 1/38* (2006.01)

(52) Cooperative Patent Classification (CPC):
**A61M 1/382; A61M 1/3696;** A61M 1/3496;
A61M 1/3609; A61M 1/362265; A61M 1/3623;
A61M 2205/331; A61M 2205/3368

(54) **SYSTEMS AND METHODS FOR UPDATING BLOOD SEPARATION CONTROL PARAMETERS USING BLOOD AND/OR BLOOD SOURCE CHARACTERISTICS**

SYSTEME UND VERFAHREN ZUR AKTUALISIERUNG VON BLUTTRENNUNGSSTEUERUNGSPARAMETERN UNTER VERWENDUNG VON BLUT- UND/ODER BLUTQUELLENEIGENSCHAFTEN

SYSTÈMES ET PROCÉDÉS DE MISE À JOUR DE PARAMÈTRES DE COMMANDE DE SÉPARATION DE SANG À L'AIDE DE CARACTÉRISTIQUES DE SOURCE SANGUINE ET/OU DE SANG

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **15.07.2022   US 202263389426 P**

(43) Date of publication of application:
**17.01.2024   Bulletin 2024/03**

(73) Proprietor: **Fenwal, Inc.**
**Lake Zurich, IL 60047 (US)**

(72) Inventor: **KUSTERS, Benjamin E.**
**Lake Zurich, 60047 (US)**

(74) Representative: **Maikowski & Ninnemann
Patentanwälte Partnerschaft mbB
Postfach 15 09 20
10671 Berlin (DE)**

(56) References cited:
**EP-A1- 3 015 126      US-A1- 2019 201 916**

## Description

Background

Field of the Disclosure

The present disclosure relates to systems and methods for controlling the position of an interface between separated blood components within a centrifuge. More particularly, the present disclosure relates to updating interface position control parameters using blood and/or blood source characteristics.

Description of Related Art

A wide variety of blood processing systems are presently in practice and allow for a blood to be fractionated or separated into its constituent parts, thus making it possible to collect particular blood constituents, rather than whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor or patient, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Whole blood is typically separated into its constituents through centrifugation. In continuous processes, this requires that the whole blood be passed through a centrifuge after it is withdrawn from, and before it is returned to, the blood source. To avoid contamination and possible infection (if the blood source is a human donor or patient), the blood is preferably contained within a preassembled, sterile fluid flow circuit or system during the entire centrifugation process. Typical blood processing systems thus include a permanent, reusable module or assembly containing the durable hardware (centrifuge, drive system, pumps, valve actuators, programmable controller, and the like) that controls the processing of the blood and blood components through a disposable, sealed, and sterile flow circuit that includes a centrifugation chamber and is mounted in cooperation on the hardware.

The hardware engages and spins the disposable centrifugation chamber during a blood separation step. As the flow circuit is spun by the centrifuge, the heavier (greater specific gravity) components of the whole blood in the flow circuit, such as red blood cells, move radially outwardly away from the center of rotation toward the outer or "high-G" wall of the centrifugation chamber. The lighter (lower specific gravity) components, such as plasma, migrate toward the inner or "low-G" wall of the centrifuge. Various ones of these components can be selectively removed from the whole blood by providing appropriately located outlet ports in the flow circuit.

Detection and control of the location of the interface between separated blood components (typically, between red blood cells and plasma) within the centrifugation chamber is important for efficient collection of the separated components. The interface control systems of conventional blood processing systems apply optical-based methods and proportional-integral-derivative ("PID") control logic to establish and maintain the interface at the target position. More particularly, a PID control algorithm is employed to minimize the difference between the target position of the interface and the actual or current position of the interface by considering the present difference between the two interface positions ("the P term"), how long and how far the interface position has been from the target position in the past ("the I term"), and a rate of change of the interface position between two time points ("the D term"). The use of PID control algorithm for controlling the location of said interface is for example described in US 2019/0201916 or EP 3 015 126 A1.

In all known predicate systems, the parameters of the PID control algorithm (e.g., the PID gains) remain constant for all separation procedures and blood characteristics. While the use of constant parameters has proven to be effective, it has been found that conventional systems experience control instability at times, particularly when characteristics of the blood (e.g., hematocrit or inflow rate) change. Thus, it would be advantageous to provide systems and methods in which the parameters of a PID control algorithm are informed by blood and/or blood source characteristics, including parameters of the PID control algorithm being updated during a procedure.

Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood separation device includes a centrifugal separator, a pump system, an interface monitoring assembly, and a controller. The controller is configured to actuate the pump system to pump blood having a plurality of blood characteristics from a blood source having a plurality of source characteristics into the centrifugal separator at an inflow rate. The controller actuates the centrifugal separator to separate the blood in the centrifugal separator into two blood components, with an interface therebetween, along with actuating the pump system to pump one of the blood components out of the centrifugal separator at an outflow rate. The controller actuates the interface monitoring assembly to determine a current position of the interface within the centrifugal separator, with the controller comparing the current position of the interface to a target interface position and generating an error signal indicative of a distance between the current position of the interface and the target interface position. The error signal is input into a proportional-integral-derivative control algorithm to generate a control signal. The controller then repeats this process, with the outflow rate being modified based at least in part on the control signal and with the proportional-integral-derivative control algorithm being modified when a value of at least one of the blood characteristics, at least one of the source characteristics, and/or the inflow rate when executing the process is different from the corresponding value when most recently executing the process.

In another aspect, a method is provided for separating blood. The method includes conveying blood having a plurality of blood characteristics from a blood source having a plurality of source characteristics into a centrifugal separator at an inflow rate. The blood in the centrifugal separator is separated into two blood components, with an interface therebetween. One of the blood components is conveyed out of the centrifugal separator at an outflow rate. A current position of the interface is determined and compared to a target interface position, with an error signal (which is indicative of a distance between the current position of the interface and the target interface position) being generated. The error signal is inputted into a proportional-integral-derivative control algorithm to generate a control signal. The process is then repeated, with the outflow rate being modified based at least in part on the control signal and with the proportional-integral-derivative control algorithm being modified when a value of at least one of the blood characteristics, at least one of the source characteristics, and/or the inflow rate when executing the process is different from the corresponding value when most recently executing the process.

Brief Description of the Drawings

Fig. 1 is a perspective view of an exemplary blood separation device according to an aspect of the present disclosure;
Fig. 2 is a schematic view of an exemplary disposable fluid flow circuit that may be mounted to the blood separation device of Fig. 1 to complete a blood separation system;
Fig. 3 is a perspective view of an exemplary centrifugal separator of the blood separation device of Fig. 1, with the centrifugal separation chamber of a fluid flow circuit mounted therein;
Fig. 4 is a top plan view of an exemplary cassette of a fluid flow circuit, which can be actuated to perform a variety of different blood processing procedures in association with the blood separation device shown in Fig. 1;
Fig. 5 is a perspective view of the centrifugal separator of Fig. 3, with selected portions thereof broken away to show a light source of an interface monitoring system;
Fig. 6 is a perspective view of the centrifugal separator of Fig. 3, with the light source operating to transmit a light beam to a light detector of the interface monitoring system;
Fig. 7 is a perspective view of the centrifugal separator of Fig. 3, with selected portions thereof broken away to show the light source and light detector of the interface monitoring system;
Fig. 8 is a perspective view of an exemplary centrifugal separation chamber of a fluid flow circuit;
Fig. 9 is a front elevational view of the centrifugal separation chamber of Fig. 8;
Fig. 10 is an enlarged perspective view of a portion of a channel of the centrifugal separation chamber of Figs. 8 and 9, with an interface between separated blood components being positioned at a desired or target location on a ramp defined within the channel;
Fig. 11 is an enlarged perspective view of the channel of Fig. 10, with the interface being at an undesired high location on the ramp;
Fig. 12 is an enlarged perspective view of the channel of Fig. 10, with the interface being at an undesired low location on the ramp;
Fig. 13 is a perspective view of a prismatic reflector used in combination with the centrifugal separation chamber of Figs. 8 and 9;
Fig. 14 is a perspective view of the prismatic reflector of Fig. 13, showing light being transmitted therethrough;
Figs. 15-18 are diagrammatic views of the ramp and prismatic reflector of the centrifugal separation chamber passing through the path of light from the light source during a calibration phase;
Figs. 19-22 are diagrammatic views of the voltage output or signal transmitted by the light detector during the conditions shown in Figs. 15-18, respectively;

Figs. 23-26 are diagrammatic views of the ramp and prismatic reflector passing through the path of light from the light source during a separation procedure;

Figs. 27-30 are diagrammatic views of the voltage output or signal transmitted by the light detector during the conditions shown in Figs. 23-26, respectively;

Figs. 31 and 32 are diagrammatic views of separated blood components on the ramp and the pulse widths of a signal generated by the light detector for each condition;

Fig. 33 is a diagrammatic view of saline on the ramp and the pulse width of a signal generated by the light detector for such a condition;

Fig. 34 is a diagrammatic view of the position of an interface between separated blood components on the ramp compared to a target interface position;

Fig. 35 is a diagrammatic view of a conventional approach to controlling the position of an interface between separated blood components;

Fig. 36 is a diagrammatic view of a conventional proportional-integral-derivative control algorithm implemented to control interface position during a blood separation procedure;

Fig. 37 is a diagrammatic view of an approach to controlling the position of an interface between separated blood components according to an aspect of the present disclosure; and

Figs. 38-41 illustrate an exemplary implementation of the interface control protocol of Fig. 37.

Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

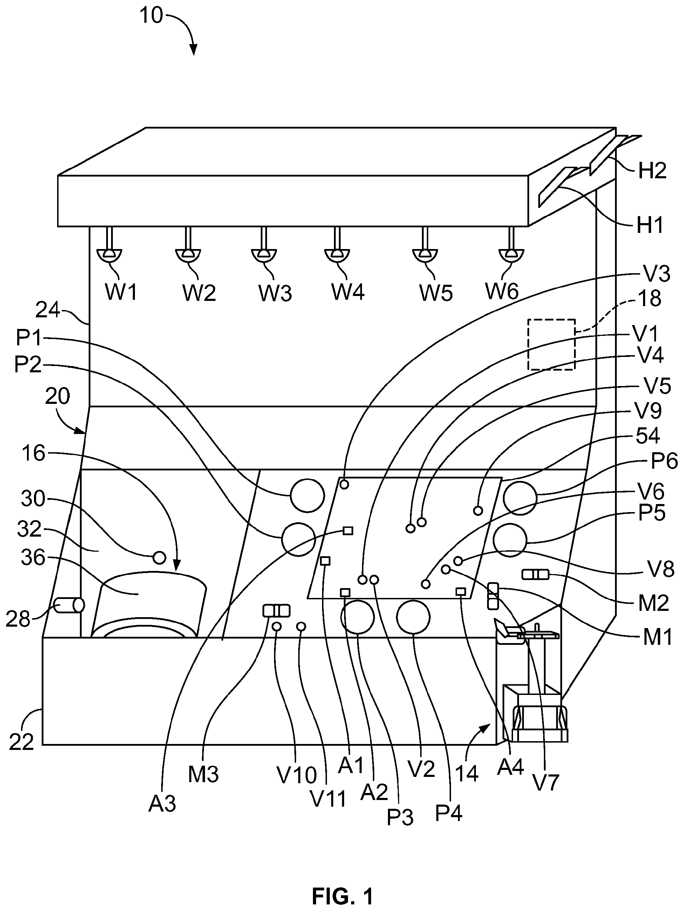

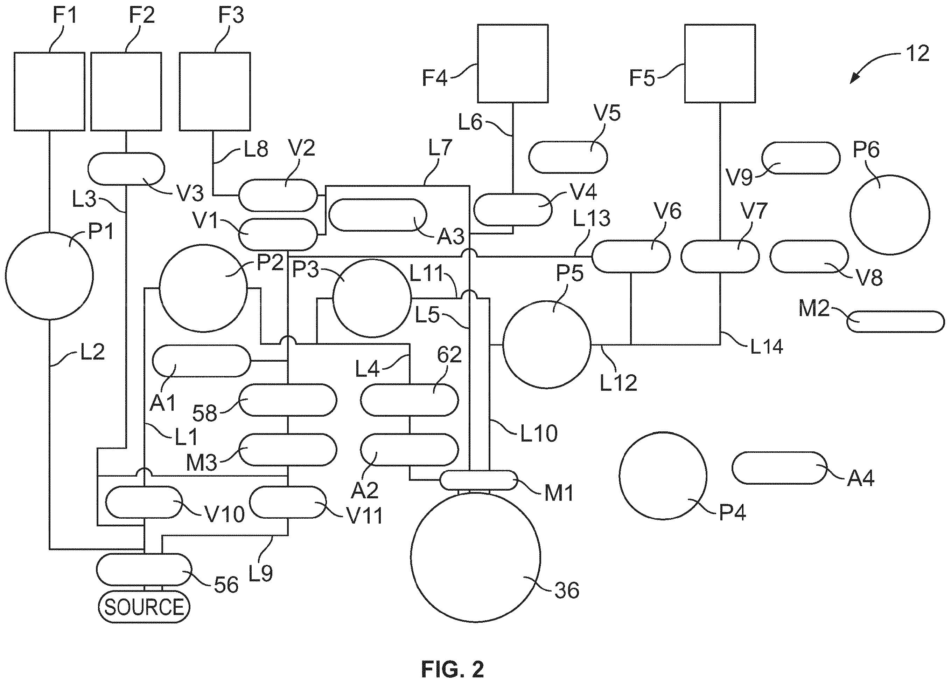

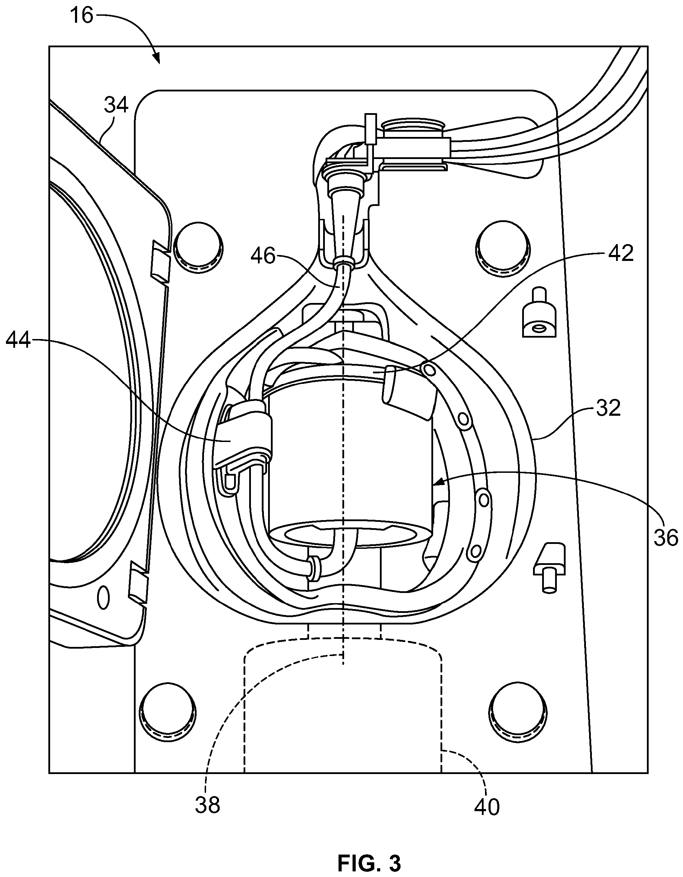

Figs. 1-41 show components of a blood or fluid separation system that embodies various aspects of the present subject matter. Generally speaking, the system includes two principal components, a durable and reusable blood separation device 10 (Fig. 1) and a disposable fluid flow circuit 12 (Fig. 2). The blood separation device 10 includes a spinning membrane separator drive unit 14 (Fig. 1), a centrifuge or centrifugal separator 16 (Fig. 3), additional components that control fluid flow through the disposable flow circuit 12, and a controller 18 (Fig. 1), which governs the operation of the other components of the blood separation device 10 to perform a blood processing and collection procedure selected by an operator.

I. The Durable Blood Separation Device

The blood separation device 10 (Fig. 1) is configured as a durable item that is capable of long-term use. It should be understood that the blood separation device 10 of Fig. 1 is merely exemplary of one possible configuration and that blood separation devices according to the present disclosure may be differently configured. For example, it is within the scope of the present disclosure for the blood separation device to omit a spinning membrane separator drive unit 14 and to include only a centrifugal separator 16, as in the blood separation device described in PCT Patent Application Publication No. WO 2021/194824 A1.

In the illustrated embodiment, the blood separation device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24. The configuration and operation of the centrifugal separator 16 and selected other components of the fluid separation device 10 will be described in greater detail.

In the illustrated embodiment, the generally horizontal portion 22 is intended to rest on an elevated, generally horizontal support surface (e.g., a countertop or a tabletop), but it is also within the scope of the present disclosure for the case 20 to include a support base to allow the case 20 to be appropriately positioned and oriented when placed onto a floor or ground surface. It is also within the scope of the present disclosure for the case 20 to be mounted to a generally vertical surface (e.g., a wall), by either fixedly or removably securing the generally vertical portion 24 of the case 20 to the surface.

The case 20 may be configured to assume only the position or configuration of Fig. 1 or may be configured to move between two or more positions or configurations. For example, in one embodiment, the generally horizontal and vertical portions 22 and 24 are joined by a hinge or pivot, which allows the case 20 to be moved between a functional or open configuration (Fig. 1) in which the generally vertical portion 24 is oriented at approximately 90 degrees to the generally horizontal portion 22 and a transport or closed configuration in which the generally vertical portion 24 is rotated about the hinge to approach the generally horizontal portion 22. In such a reconfigurable embodiment, the generally vertical portion 24 may be considered to be the lid of the case 20, while the generally horizontal portion 22 may be considered to be the base. If the case 20 is so reconfigurable, then it may include a latch for releasably locking the case 20 in its closed configuration and/or a handle, which may be grasped for transporting the case 20 in its closed configuration.

While it may be advantageous for the blood separation device 10 to be embodied in a compact, portable case 20, it is also within the scope of the present disclosure for the blood separation device to be embodied in a larger case or fixture that is intended to be installed in a single location and remain in that location for an extended period of time. If the blood separation device is provided as a fixture, it may be provided with more components and functionality than a more portable version.

## A. Centrifugal Separator

The centrifugal separator 16 includes a centrifuge compartment 32 that may receive the other components of the centrifugal separator 16 (Fig. 3). The centrifuge compartment 32 may include a lid 34 that is opened to insert and remove a centrifugal separation chamber 36 of the fluid flow circuit 12. During a separation procedure, the lid 34 may be closed with the centrifugal separation chamber 36 positioned within the centrifuge compartment 32, as the centrifugal separation chamber 36 is spun or rotated about an axis 38 under the power of an electric drive motor or rotor 40 of the centrifugal separator 16.

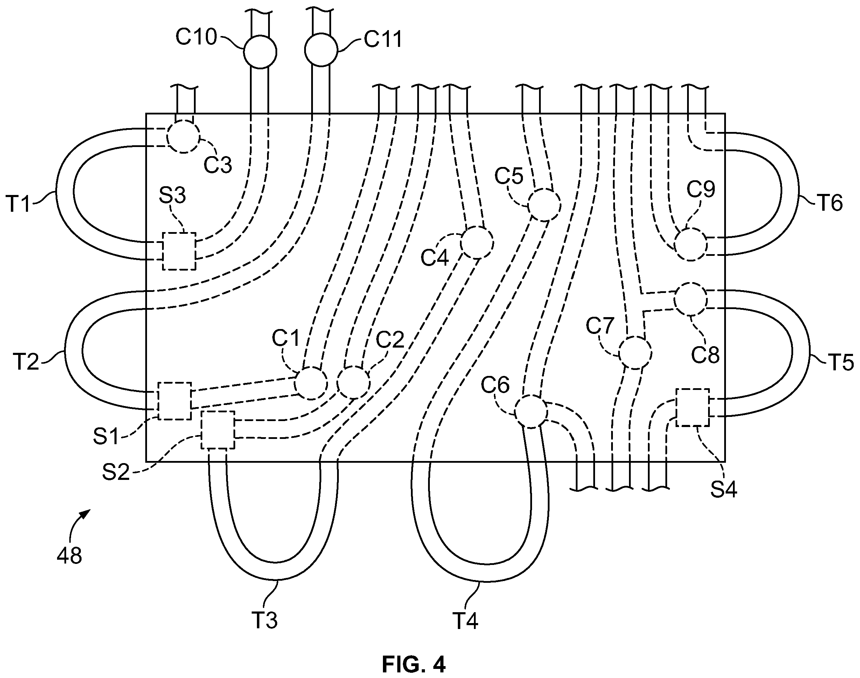

The particular configuration and operation of the centrifugal separator 16 depends upon the particular configuration of the centrifugal separation chamber 36 of the fluid flow circuit 12. In one embodiment, the centrifugal separator 16 is similar in structure and operation to that of the ALYX® system manufactured by Fenwal, Inc. of Lake Zurich, Illinois, which is an affiliate of Fresenius Kabi AG of Bad Homburg, Germany, as described in greater detail in U.S. Patent No. 8,075,468. More particularly, the centrifugal separator 16 may include a carriage or support 42 that holds the centrifugal separation chamber 36 and a yoke member 44. The yoke member 44 engages an umbilicus 46 of the fluid flow circuit 12, which extends between the centrifugal separation chamber 36 and a cassette 48 of the fluid flow circuit 12 (Fig. 4). The yoke member 44 causes the umbilicus 46 to orbit around the centrifugal separation chamber 36 at a one omega rotational speed. The umbilicus 46 twists about its own axis as it orbits around the centrifugal separation chamber 36. The twisting of the umbilicus 46 about its axis as it rotates at one omega with the yoke member 44 imparts a two omega rotation to the centrifugal separation chamber 36, according to known design. The relative rotation of the yoke member 44 at a one omega rotational speed and the centrifugal separation chamber 36 at a two omega rotational speed keeps the umbilicus 46 untwisted, avoiding the need for rotating seals.

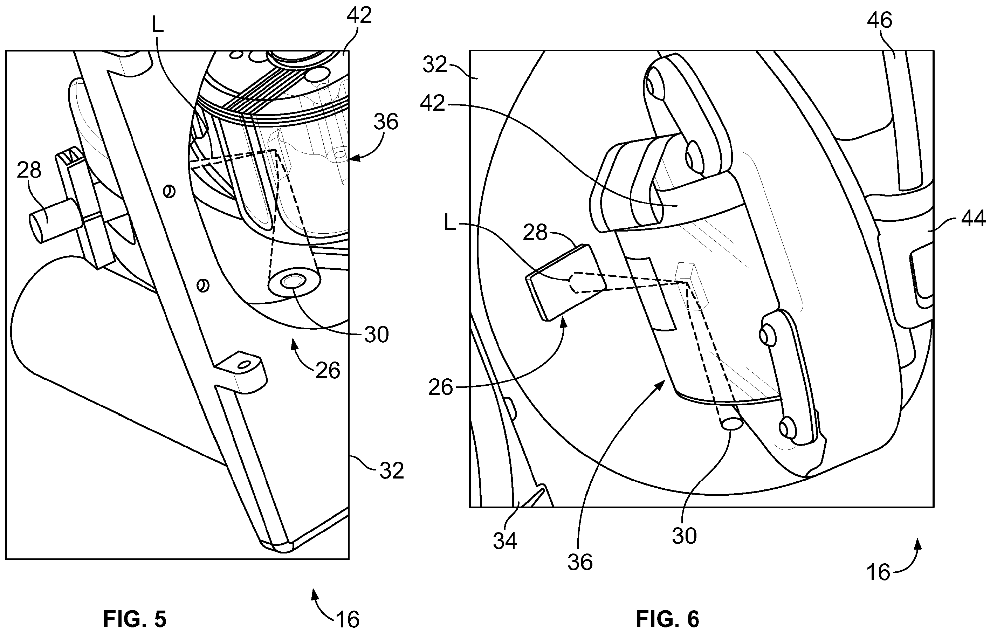

Blood is introduced into the centrifugal separation chamber 36 by the umbilicus 46, with the blood being separated (e.g., into a layer of less dense components, such as platelet-rich plasma, and a layer of more dense components, such as packed red blood cells) within the centrifugal separation chamber 36 as a result of centrifugal forces as it rotates. Components of an interface monitoring assembly 26 (which will be described in greater detail herein) may be positioned within the centrifuge compartment 32 to oversee separation of blood within the centrifugal separation chamber 36. As shown in Figs. 5-7, the interface monitoring assembly 26 may include a light source 28 and a light detector 30, which is positioned and oriented to receive at least a portion of the light emitted by the light source 28. The light source 28 emits a light beam through the separated blood components within the centrifugal separation chamber 36 (which may be formed of a material that substantially transmits the light or at least a particular wavelength of the light without absorbing it). A portion of the light reaches the light detector 30, which transmits a signal to the controller 18 that is indicative of the location of an interface between the separated blood components. If the controller 18 determines that the interface is not at a target location (which can affect the separation efficiency of the centrifugal separator 16 and/or the quality of the separated blood components), then it can issue commands to the appropriate components of the blood separation device 10 to modify their operation so as to move the interface to the proper location.

## B. Other Components Of The Fluid Separation Device

In addition to the spinning membrane separator drive unit 14 and the centrifugal separator 16, the blood separation device 10 may include other components compactly arranged to aid blood processing.

The generally horizontal portion 22 of the case 20 of the illustrated blood separation device 10 includes a cassette station 54, which accommodates a cassette 48 of the fluid flow circuit 12 (Fig. 4). In one embodiment, the cassette station 54 is similarly configured to the cassette station of U.S. Patent No. 5,868,696 , but is adapted to include additional components and functionality. The illustrated cassette station 54 includes a plurality of clamps or valves V1-V9 (Fig. 1), which move between a plurality of positions (e.g., between a retracted or lowered position and an actuated or raised position) to selectively contact or otherwise interact with corresponding valve stations C1-C9 of the cassette 48 of the fluid flow circuit 12 (Fig. 4). Depending on the configuration of the fluid flow circuit 12, its cassette 48 may not include a valve station C1-C9 for each valve V1-V9 of the cassette station 54, in which case fewer than all of the valves V1-V9 will be used in a separation procedure.

In the actuated position, a valve V1-V9 engages the associated valve station C1-C9 to prevent fluid flow through that valve station C1-C9 (e.g., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (e.g., by opening one or more ports associated with the valve station C1-C9, thereby allowing fluid flow through that port or ports). Additional clamps or valves V10 and V11 may be positioned outside of the cassette station 54 to interact with portions or valve stations C10 and C11 (which may be lengths of tubing) of the fluid flow circuit 12 to selectively allow and prevent fluid flow therethrough. The valves V1-V9 and corresponding valve stations C1-C9 of the cassette station 54 and cassette 48 may be differently configured and operate differently from the valves V10 and V11 and valve stations C10 and C11 that are spaced away from the cassette station 54.

The cassette station 54 may be provided with additional components, such as pressure sensors A1-A4, which interact with sensor stations S1-S4 of the cassette 48 to monitor the pressure at various locations of the fluid flow circuit 12. For example, if the blood source is a human patient, one or more of the pressure sensors A1-A4 may be configured to monitor the pressure of the patient's vein during blood draw and return. Other pressure sensors A1-A4 may monitor the pressure of a spinning membrane separator received by the spinning membrane separator drive unit 14 and a centrifugal separation chamber 36 received by the centrifugal separator 16. The controller 18 may receive signals from the pressure sensors A1-A4 that are indicative of the pressure within the fluid flow circuit 12 and, if a signal indicates a low- or high-pressure condition, the controller 18 may initiate an alarm or error condition to alert an operator to the condition and/or to attempt to bring the pressure to an acceptable level without operator intervention.

The blood separation device 10 may also include a plurality of pumps P1-P6 (which may be collectively referred to as a pump assembly or pump system) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop T1-T6 extending from a side surface of the cassette 48 (Fig. 4) and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops T1-T6 into the associated pump P1-P6.

The illustrated blood separation device 10 also includes a centrifugal separator sensor M1 for determining one or more properties of fluids flowing out of and/or into the centrifugal separator 16. If the fluid flowing out of the centrifugal separator 16 includes red blood cells, the centrifugal separator sensor M1 may be configured to determine the hematocrit of the fluid. If the fluid flowing out of the centrifugal separator 16 is platelet-rich plasma, the centrifugal separator sensor M1 may be configured to determine the platelet concentration of the platelet-rich plasma. The centrifugal separator sensor M1 may detect the one or more properties of a fluid by optically monitoring the fluid as it flows through tubing of the fluid flow circuit 12 or by any other suitable approach. The controller 18 may receive signals from the centrifugal separator sensor M1 that are indicative of the one or more properties of fluid flowing out of the centrifugal separator 16 and use the signals to optimize the separation procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 , but it should be understood that a different approach may also be employed for monitoring hematocrit and/or platelet concentration of fluid flowing out of the centrifugal separator 16.

The illustrated blood separation device 10 further includes a spinner outlet sensor M2, which accommodates tubing of a fluid flow circuit that flows a separated blood component out of a spinning membrane separator received by the spinning membrane separator drive unit 14. The fluid flow circuit 12 of Fig. 2 does not employ a spinning membrane separator, so the spinner outlet sensor M2 is not described in detail herein.

The illustrated fluid separation device 10 also includes an air detector M3 (e.g., an ultrasonic bubble detector), which accommodates tubing of the fluid flow circuit 12 that flows fluid to a recipient. It may be advantageous to prevent air from reaching the recipient, so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 18 may include a plurality of weight scales W1-W6 (six are shown, but more or fewer may be provided), each of which may support one or more fluid containers F1-F5 of the fluid flow circuit 12 (Fig. 2). The containers F1-F5 receive fluid components or waste products separated during processing or intravenous fluids or additive fluids. Each weight scale W1-W6 transmits to the controller 18 a signal that is indicative of the weight of the fluid within the associated container F1-F5 to track the change of weight during the course of a procedure. This allows the controller 18 to process the incremental weight changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

C. Controller

According to an aspect of the present disclosure, the blood separation device 10 includes a controller 18, which is suitably configured and/or programmed to control operation of the blood separation device 10. In one embodiment, the controller 18 comprises a main processing unit (MPU), which can comprise, e.g., a Pentium™ type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the blood separation device 10.

The controller 18 is configured and/or programmed to execute at least one blood processing application but, more advantageously, is configured and/or programmed to execute a variety of different blood processing applications. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, a platelet/plasma collection procedure, and a mononuclear cell collection procedure. Additional or alternative procedure applications can be included without departing from the scope of the present disclosure.

More particularly, in carrying out any one of these blood processing applications, the controller 18 is configured and/or programmed to control one or more of the following tasks: drawing blood into a fluid flow circuit 12 mounted to the blood separation device 10, conveying blood through the fluid flow circuit 12 to a location for separation (i.e., into a spinning membrane separator or centrifugal separation chamber 36 of the fluid flow circuit 12), separating the blood into two or more components as desired, and conveying the separated components into a storage container or to a recipient (which may be the source from which the fluid was originally drawn).

This may include instructing the spinning membrane separator drive unit 14 or the centrifugal separator 16 to operate at a particular rotational speed and instructing a pump P1-P6 to convey fluid through a portion of the fluid flow circuit 12 at a particular flow rate. Hence, while it may be described herein that a particular component of the blood separation device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the blood separation device 10 (e.g., the pressure sensors A1-A4) to monitor various aspects of the operation of the blood separation device 10 and characteristics of the blood and separated blood components as they flow through the fluid flow circuit 12. If the operation of any of the components and/or one or more characteristics of the blood or separated blood components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 30 of the interface monitoring assembly 26. The signals that the controller 18 receives from the light detector 30 are indicative of the location of an interface between the separated blood components within the centrifugal separation chamber 36. If the controller 18 determines that the interface is not at a target location, then it can issue commands to the appropriate components of the blood separation device 10 to modify their operation so as to move the interface to the proper location. For example, the controller 18 may instruct one of the pumps P1-P6 to cause blood to flow into the centrifugal separation chamber 36 at a different rate and/or for a separated blood component to be removed from the centrifugal separation chamber 36 at a different rate and/or for the centrifugal separation chamber 36 to be spun at a different speed by the centrifugal separator 16. A particular protocol carried out by the interface control module in adjusting the position of the interface within the centrifugal separation chamber 36 will be described in greater detail.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

## II. The Disposable Fluid Flow Circuit

### A. Overview

As for the fluid flow circuit or flow set 12 (Fig. 2), it is intended to be a sterile, single use, disposable item. Before beginning a given blood separation procedure, the operator loads various components of the fluid flow circuit 12 in the case 20 in association with the blood separation device 10. The controller 18 implements the procedure based upon preset protocols, taking into account other input from the operator. Upon completing the procedure, the operator removes the fluid flow circuit 12 from association with the blood separation device 10. The portions of the fluid flow circuit 12 holding the collected blood component or components (e.g., collection containers or bags) are removed from the case 20 and retained for storage, transfusion, or further processing. The remainder of the fluid flow circuit 12 is removed from the case 20 and discarded.

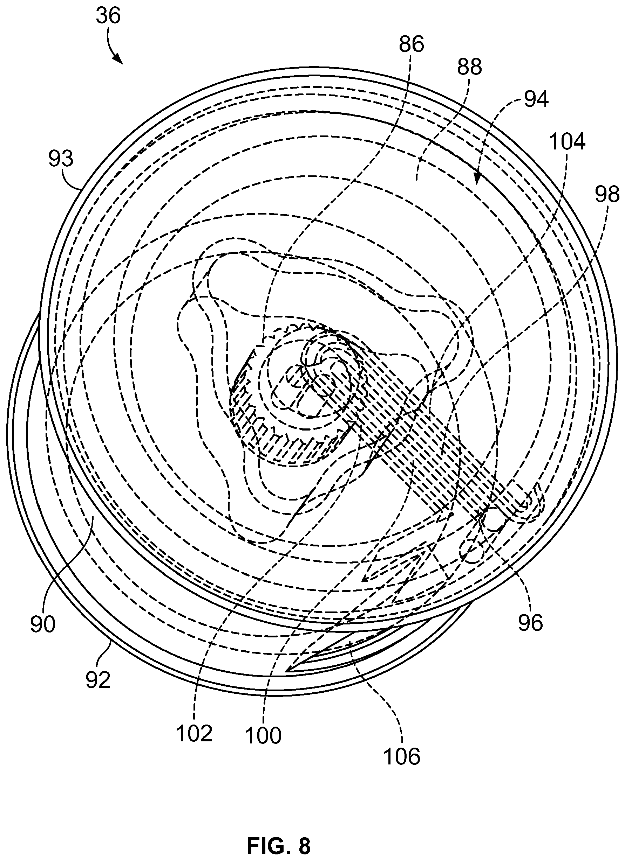

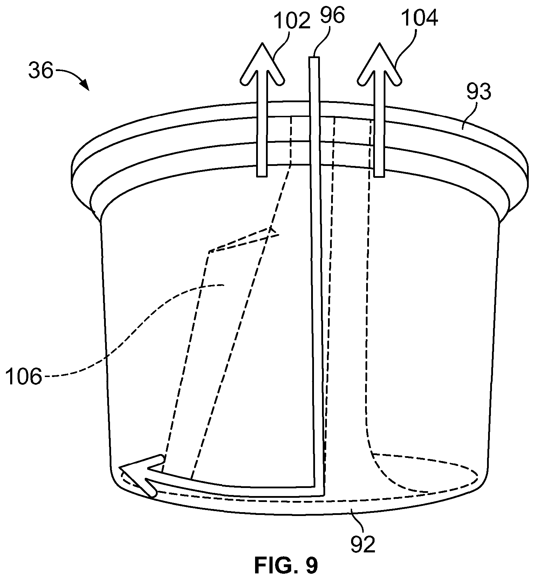

A variety of different disposable fluid flow circuits may be used in combination with the blood separation device 10, with the appropriate fluid flow circuit depending on the separation procedure to be carried out using the system. Generally speaking, though, the fluid flow circuit 12 includes a cassette 48 (Fig. 4), to which the other components of the fluid flow circuit 12 are connected by flexible tubing. The other components may include a plurality of fluid containers F1-F5 (for holding blood, a separated blood component, an intravenous fluid, or an additive solution, for example), one or more fluid source access devices (e.g., a connector for accessing blood within a fluid container), and a spinning membrane separator (not illustrated) and/or a centrifugal separation chamber 36 (Figs. 8 and 9).

### B. Cassette And Tubing

The cassette 48 (Fig. 4) provides a centralized, programmable, integrated platform for all the pumping and many of the valving functions required for a given blood processing procedure. In one embodiment, the cassette 48 is similarly configured to the cassette of U.S. Patent No. 5,868,696, but is adapted to include additional components (e.g., more tubing loops T1-T6) and functionality.

In use, the cassette 48 is mounted to the cassette station 54 of the blood separation device 10, with a flexible diaphragm of the cassette 48 placed into contact with the cassette station 54. The flexible diaphragm overlays an array of interior cavities formed by the body of the cassette 48. The different interior cavities define sensor stations S1-S4, valve stations C1-C9, and a plurality of flow paths. The side of the cassette 48 opposite the flexible diaphragm may be sealed by another flexible diaphragm or a rigid cover, thereby sealing fluid flow through the cassette 48 from the outside environment.

Each sensor station S1-S4 is aligned with an associated pressure sensor A1-A4 of the cassette station 54, with each pressure sensor A1-A4 capable of monitoring the pressure within the associated sensor station S1-S4. Each valve station C1-C9 is aligned with an associated valve V1-V9, and may define one or more ports that allow fluid communication between the valve station C1-C9 and another interior cavity of the cassette 48 (e.g., a flow path). As described above, each valve V1-V9 is movable under command of the controller 18 to move between a plurality of positions (e.g., between a retracted or lowered position and an actuated or raised position) to selectively contact the valve stations C1-C9 of the cassette 48. In the actuated position, a valve V1-V9 engages the associated valve station C1-C9 to close one or more of its ports to prevent fluid flow therethrough. In the retracted position, a valve V1-V9 is disengaged from the associated valve station C1-C9 (or less forcefully contacts the associated valve station C1-C9 than when in the actuated position) to open one or more ports associated with the valve station C1-C9, thereby allowing fluid flow therethrough.

As described, a plurality of tubing loops T1-T6 extend from the side surface of the cassette 48 to interact with pumps P1-P6 of the blood separation device 10. In the illustrated embodiment, six tubing loops T1-T6 extend from the cassette 48 to be received by a different one of six pumps P1-P6, but in other embodiments, a procedure may not require use of all of the pumps P1-P6, in which case the cassette 48 may include fewer than six tubing loops. The different pumps P1-P6 may interact with the tubing loops T1-T6 of the cassette 48 to perform different tasks during a separation procedure. Certain procedures require fewer than all of the sensor stations, valve stations, and/or tubing loops illustrated in the exemplary cassette 48 of Fig. 4, such that it should be understood that the cassettes of different fluid flow circuits 12 may be differently configured (e.g., with fewer sensor stations, valve stations, and/or tubing loops) without departing from the scope of the present disclosure.

Additional tubing extends from the side surface of the cassette 48 to connect to the other components of the fluid flow circuit 12, such as the various fluid containers F1-F5, a spinning membrane separator (if provided), and the centrifugal separation chamber 36. The tubing connected to the centrifugal separation chamber 36 (which includes one inlet tube and two outlet tubes) may be aggregated into an umbilicus 46 (Fig. 3) that is engaged by the yoke member 44 of the centrifugal separator 16 (as described above) to cause the umbilicus 46 to orbit around and spin or rotate the centrifugal separation chamber 36 during a separation procedure.

Various additional components may be incorporated into the tubing leading out of the cassette 48 or into one of the cavities of the cassette 48. For example, as shown in Fig. 2, a manual clamp 56 may be associated with a line or lines leading to the fluid source and/or fluid recipient, a return line filter 58 (e.g., a microaggregate filter) may be associated with a line leading to a fluid recipient, filters may be positioned upstream of one or more of the fluid containers to remove a substance (e.g., leukocytes) from a separated component (e.g., red blood cells or platelets) flowing into the fluid container, and/or an air trap 62 may be positioned on a line upstream of the centrifugal separation chamber 36.

C. Centrifugal Separation Chamber

The fluid flow circuit 12 is provided with a centrifugal separation chamber 36, with Figs. 8 and 9 illustrating an exemplary centrifugal separation chamber 36. In the illustrated embodiment, the body of the centrifugal separation chamber 36 is pre-formed in a desired shape and configuration (e.g., by injection molding) from a rigid, biocompatible plastic material, such as a non-plasticized medical grade acrylonitrile-butadiene-styrene (ABS). All contours, ports, channels, and walls that affect the fluid separation process are preformed in a single, injection molded operation. Alternatively, the centrifugal separation chamber 36 can be formed by separate molded parts, either by nesting cup-shaped subassemblies or two symmetric halves.

The underside of the centrifugal separation chamber 36 includes a shaped receptacle 86 (Fig. 8) that is suitable for receiving an end of the umbilicus 46 of the fluid flow circuit 12 (Fig. 3). A suitable receptacle 86 and the manner in which the umbilicus 46 may cooperate with the receptacle 86 to deliver fluid to and remove fluid from the centrifugal separation chamber 36 are described in greater detail in U.S. Patent No. 8,075,468.

The illustrated centrifugal separation chamber 36 has radially spaced apart inner (low-g) and outer (high-g) side wall portions 88 and 90, a bottom or first end wall portion 92, and a cover or second end wall portion 93. The cover 93 comprises a simple flat part that can be easily welded or otherwise secured to the body of the centrifugal separation chamber 36. Because all features that affect the separation process are incorporated into one injection molded component, any tolerance differences between the cover 93 and the body of the centrifugal separation chamber 36 will not affect the separation efficiencies of the centrifugal separation chamber 36. The wall portions 88 and 90, the bottom 92, and the cover 93 together define an enclosed, generally annular channel 94.

The (whole blood) inlet 96 communicating with the channel 94 is defined between opposing interior radial walls 98 and 100. One of the interior walls 98 joins the outer (high-g) wall portion 90 and separates the upstream and downstream ends of the channel 94. The interior walls 98 and 100 define the inlet passageway 96 of the centrifugal separation chamber 36 which, in one flow configuration, allows fluid to flow from the umbilicus 46 to the upstream end of the channel 94.

The illustrated centrifugal separation chamber 36 further includes first and second outlets 102 and 104, respectively, which may be defined by opposing surfaces of interior radial walls. Both the first and second outlets 102 and 104 extend radially inward from the channel 94. The first (plasma) outlet 102 extends radially inward from an opening which, in the illustrated embodiment, is located at the inner side wall portion 88, while the second (red blood cell) outlet 104 extends radially inward from an opening that is associated with the outer side wall portion 90. The illustrated first outlet 102 is positioned adjacent to the inlet 96 (near the upstream end of the channel 94), while the second outlet 104 may be positioned at the opposite, downstream end of the channel 94.

It should be understood that the centrifugal separation chamber 36 illustrated in Figs. 8 and 9 is merely exemplary and that the centrifugal separation chamber 36 may be differently configured without departing from the scope of the present disclosure.

1. Centrifugal Separation And Interface Detection Principles

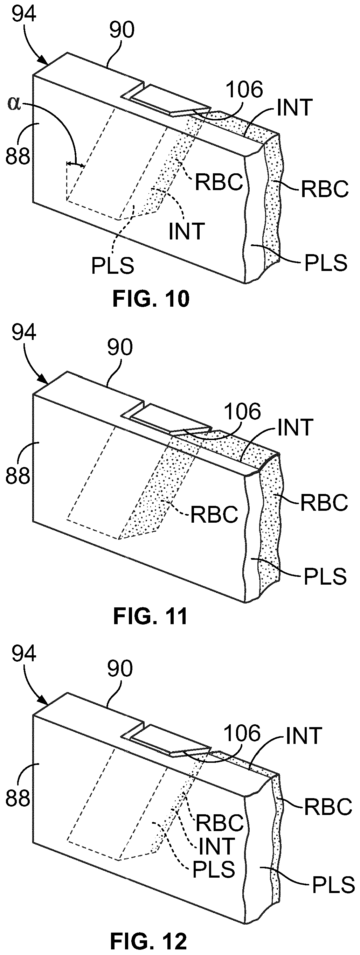

Blood flowed into the channel 94 separates into an optically dense layer RBC and a less optically dense layer PLS (Figs. 10-12) as the centrifugal separation chamber 36 is rotated about the rotational axis 38. The optically dense layer RBC forms as larger and/or heavier blood particles move under the influence of centrifugal force toward the outer (high-g) wall portion 90. The optically dense layer RBC will typically include red blood cells (and, hence, may be referred to herein as the "RBC layer") but, depending on the speed at which the centrifugal separation chamber 36 is rotated, other cellular components (e.g., larger white blood cells) may also be present in the optically dense layer RBC.

The less optically dense layer PLS typically includes a plasma constituent, such as platelet-rich plasma (and, hence, may be referred to herein as the "PLS layer"). Depending on the speed at which the centrifugal separation chamber 36 is rotated and the length of time that the blood is resident therein, other components (e.g., smaller white blood cells and anticoagulant) may also be present in the less optically dense layer PLS.

In one embodiment, blood introduced into the channel 94 via the inlet 96 will travel in a generally clockwise direction (in the orientation of Fig. 8) as the optically dense layer RBC separates from the less optically dense layer PLS. The optically dense layer RBC continues moving in the clockwise direction as it travels the length of the channel 94 along the outer side wall portion 90, from the upstream end to the downstream end, where it exits the channel 94 via the second outlet 104. The less optically dense layer PLS separated from the optically dense layer RBC reverses direction, moving counterclockwise along the inner side wall portion 88 to the first outlet 102, adjacent to the inlet 96. The inner side wall portion 88 may be tapered inward as it approaches the second outlet 104 to force the plasma liberated at or adjacent to the downstream end of the channel 94 to drag the interface back towards the upstream end of the channel 94, where the lower surface hematocrit will re-suspend any platelets settled on the interface.

As described above, the transition between the optically dense layer RBC and the less optically dense layer PLS may be referred to as the interface INT. The location of the interface INT within the channel 94 of the centrifugal separation chamber 36 can dynamically shift during blood processing, as Figs. 10-12 show. If the location of the interface INT is too high (that is, if it is too close to the inner side wall portion 88 and the first outlet 102, as in Fig. 11), red blood cells can flow into the first outlet 102, potentially adversely affecting the quality of the low-density components (platelet-rich plasma or platelet-poor plasma). On the other hand, if the location of the interface INT is too low (that is, if it resides too far away from the inner wall portion 88, as Fig. 12 shows), the collection efficiency of the system may be impaired. The ideal or target location of the interface INT may be experimentally determined, which may vary depending on any of a number of factors (e.g., the configuration of the centrifugal separation chamber 36, the rate at which the centrifugal separation chamber 36 is rotated about the rotational axis 38, etc.).

As described above, the blood separation device 10 may include an interface monitoring assembly 26 and a controller 18 with an interface control module to monitor and, as necessary, correct the location of the interface INT. In one embodiment, the centrifugal separation chamber 36 is formed with a ramp 106 extending from the high-g wall portion 90 at an angle $\alpha$ across at least a portion of the channel 94 (Figs. 8-12). The angle $\alpha$, measured with respect to the rotational axis 38 is about 25° in one embodiment. Figs. 10-12 show the orientation of the ramp 106 when viewed from the low-g side wall portion 88 of the centrifugal separation chamber 36. Although it describes a flexible separation chamber, the general structure and function of the ramp 106 may be better understood with reference to U.S. Patent No. 5,632,893. The ramp 106 may be positioned at any of a number of locations between the upstream and downstream ends of the channel 94, but in one embodiment, the ramp 106 may be positioned generally adjacent to the first outlet 102, in the path of fluid and/or a fluid component moving from the inlet 96 to the first outlet 102.

The ramp 106 makes the interface INT between the optically dense layer RBC and the less optically dense layer PLS more discernible for detection, displaying the optically dense layer RBC, less optically dense layer PLS, and interface INT for viewing through a light-transmissive portion of the centrifugal separation chamber 36. To that end, the ramp 106 and at least the portion of the centrifugal separation chamber 36 angularly aligned with the ramp 106 may be formed of a light-transmissive material, although it may be advantageous for the entire centrifugal separation chamber 36 to be formed of the same light-transmissive material.

In the illustrated embodiment, the light source 28 of the interface monitoring assembly 26 is secured to a fixture or wall of the centrifuge compartment 32 and oriented to emit a light that is directed toward the rotational axis 38 of the centrifugal separator 16, as shown in Figs. 5-7. If the light detector 30 is positioned at an angle with respect to the light source 28 (as in the illustrated embodiment), the light L emitted by the light source 28 must be redirected from its initial path before it will reach the light detector 30. In the illustrated embodiment, the light L is redirected by a reflector that is associated with a light-transmissive portion of the inner side wall portion 88, as shown in Figs. 5-7. The reflector may be a separate piece that is secured to the inner side wall portion 88 (e.g., by being bonded thereto) or may be integrally formed with the body of the centrifugal separation chamber 36.

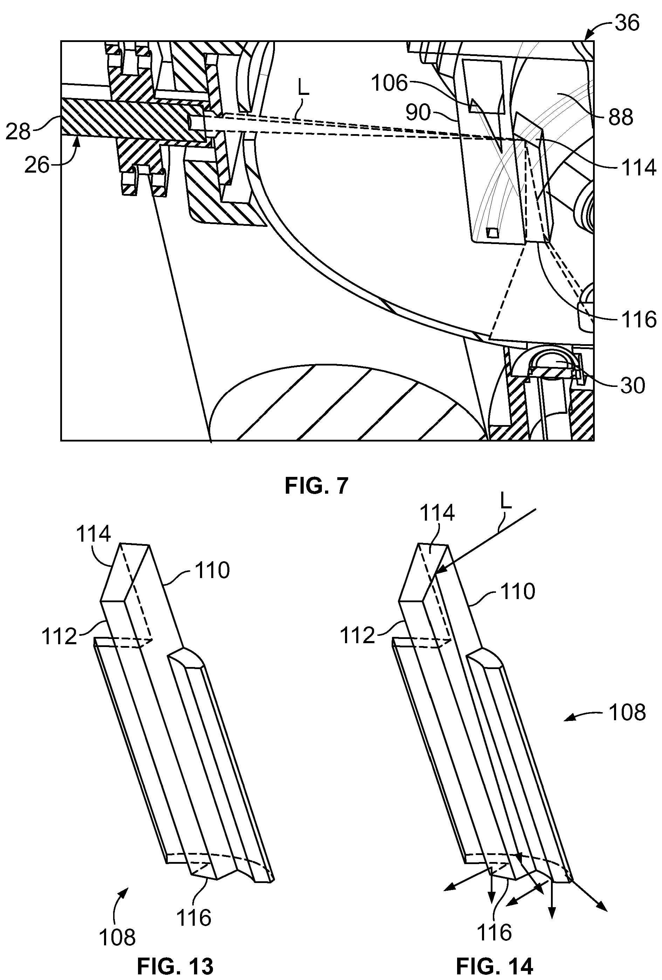

In one embodiment, the reflector may be a reflective surface, such as a mirror, that is oriented (e.g., at a 45° angle) to direct light L emitted by the light source 28 to the light detector 30. In another embodiment, the reflector is provided as a prismatic reflector 108 (Figs. 13 and 14), which is formed of a light-transmissive material (e.g., a clear plastic material) and has inner and outer walls 110 and 112 and first and second end walls 114 and 116. The inner wall 110 is positioned against the inner side wall portion 88 of the centrifugal separation chamber 36 and is oriented substantially perpendicular to the initial path of the light L from the light source 28. This allows light L from the light source 28 to enter into the prismatic reflector 108 via the inner wall 110 while continuing along its initial path. The light L continues through the prismatic reflector 108 along its initial path until it encounters the first end wall 114. The first end wall 114 is oriented at an angle (e.g., an approximately 45° angle) with respect to the first surface 110 and the second end wall 116, causing the light to be redirected within the prismatic reflector 108, rather than exiting the prismatic reflector 108 via the first end wall 114.

The first end wall 114 directs the light L at an angle to its initial path (which may be an approximately 90° angle, directing it from a path toward the rotational axis 38 to a path that is generally parallel to the rotational axis 38) toward the second end wall 116 (Fig. 14). The first end wall 114 and the inner and outer walls 110 and 112 of the prismatic reflector 108 may be configured to transmit the redirected light L from the first end wall 114 to the second end wall 116 by total internal reflection. The second end wall 116 is oriented substantially perpendicular to the redirected path of the light L through the prismatic reflector 108, such that the light L will exit the prismatic reflector 108 via the second end wall 116, continuing along its redirected path. In one embodiment, the second end wall 116 is roughened or textured or otherwise treated or conditioned to diffuse the light L as it exits the prismatic reflector 108, which may better ensure that the light L reaches the light detector 30 (Fig. 7).

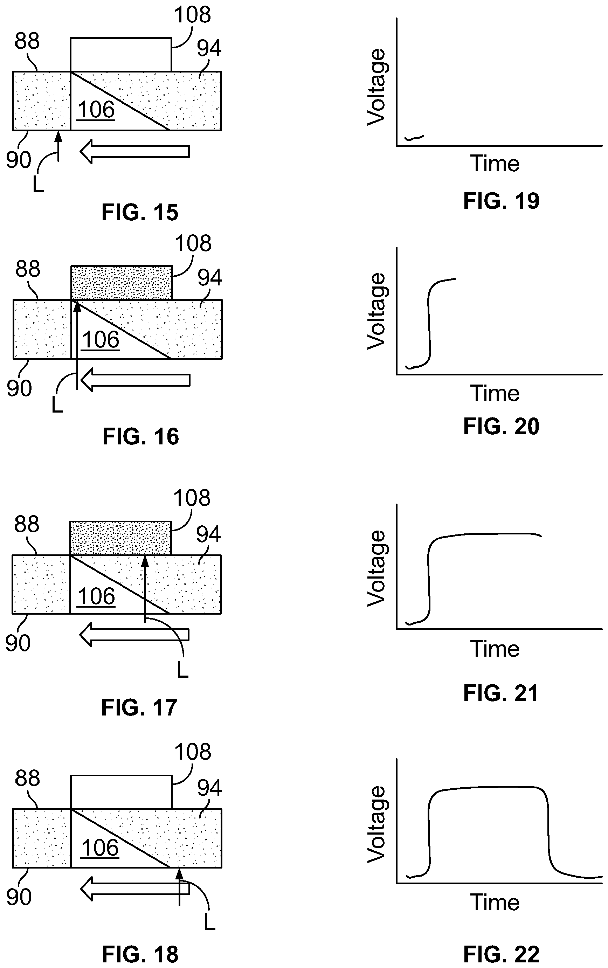

The prismatic reflector 108 may be angularly aligned with the ramp 106, such that the light L from the light source 28 will only enter into the prismatic reflector 108 when the ramp 106 has been rotated into the path of the light L. At all other times (when the ramp 106 is not in the path of the light L), the light L will not reach the prismatic reflector 108 and, thus, will not reach the light detector 30. This is illustrated in Figs. 15-18, which show the ramp 106 and prismatic reflector 108 as the centrifugal separation chamber 36 is rotated about the rotational axis 38 (while the light source 28 remains in a fixed location). In Fig. 15, the ramp 106 and prismatic reflector 108 have not yet been rotated into the initial path of the light L from the light source 28. At this time, no light is transmitted to the light detector 30, such that the output voltage of the light detector 30 (i.e., the signal transmitted from the light detector 30 to the controller 18) is in a low- or zero-state (Fig. 19).

Upon the ramp 106 first being rotated into the initial path of the light L from the light source 28 (Fig. 16), the light L will begin to reach the prismatic reflector 108, which directs the light L to the light detector 30. This causes the voltage output of the light detector 30 (i.e., the signal transmitted from the light detector 30 to the controller 18) to increase to a non-zero value or state, as shown in Fig. 20.

During a calibration phase, the channel 94 is filled with a fluid that will transmit the light L rather than absorbing or reflecting the light or otherwise preventing the light L from reaching the prismatic reflector 108, such that the voltage output of the light detector 30 will remain generally constant as the ramp 106 and prismatic reflector 108 are rotated through the initial path of the light L from the light source 28 (Figs. 17 and 21). Such a calibration phase may coincide with a priming phase during which saline is pumped through the fluid flow circuit 12 to prime the fluid flow circuit 12 or may comprise a separate phase. A calibration phase may be useful in ensuring the proper operation of the light source 28 and the light detector 30, standardizing the readings taken during a separation procedure in case of any irregularities or imperfections of the centrifugal separation chamber 36, and establishing a baseline value for the signal transmitted from the light detector 30 to the controller 18 when the ramp 106 and prismatic reflector 108 are aligned with the light source 28. As will be described in greater detail, the voltage output of the light detector 30 will typically not remain constant as the ramp 106 and prismatic reflector 108 are rotated through the initial path of the light L from the light source 28 because the different fluid layers displayed on the ramp 106 will allow different amounts of light L to reach the prismatic reflector 108.

The ramp 106 and prismatic reflector 108 are eventually rotated out of alignment with the light source 28 (Fig. 18), at which time no light L will reach the prismatic reflector 108 and the voltage output of the light detector 30 will return to its low- or zero-state (Fig. 22).

2. Detection Of Interface Position

During separation of blood within the channel 94, the light L from the light source 28 travels through a light-transmissive portion of the outer side wall portion 90 and the ramp 106 to intersect the separated blood components thereon when the ramp 106 has been rotated into the initial path of the light L. After passing through the ramp 106, the light continues through the channel 94 and the fluids in the channel 94. At least a portion of the light L (i.e., the portion not absorbed or reflected by the fluids) exits the channel 94 by striking and entering a light-transmissive portion of the inner side wall portion 88. The light L passes through the inner side wall portion 88 and enters the prismatic reflector 108, which redirects the light L from its initial path to the light detector 30, as described above.

The light detector 30 generates a signal that is transmitted to the interface control module of the controller 18, which can determine the location of the interface INT on the ramp 106. In one embodiment, the location of the interface INT is associated with a change in the amount of light L that is transmitted through the less optically dense layer PLS and the optically dense layer RBC. For example, the light source 28 may be configured to emit a light L that is more readily transmitted by platelet-rich plasma or platelet-poor plasma than by red blood cells, such as red visible light (from a laser or a differently configured light source 28), which is substantially absorbed by red blood cells. The less optically dense layer PLS and the optically dense layer RBC each occupy a certain portion of the ramp 106, with the light detector 30 receiving different amounts of light L depending on whether the light L travels through the less optically dense layer PLS on the ramp 106 or the optically dense layer RBC on the ramp 106. The percentage of the ramp 106 occupied by each layer is related to the location of the interface **INT** in the channel 94. Thus, by measuring the amount of time that the voltage output or signal from the light detector 30 is relatively high (corresponding to the time during which the light L is passing through only the less optically dense layer PLS on the ramp 106), the controller 18 may determine the location of the interface **INT** and take steps to correct the location of the interface **INT,** if necessary.

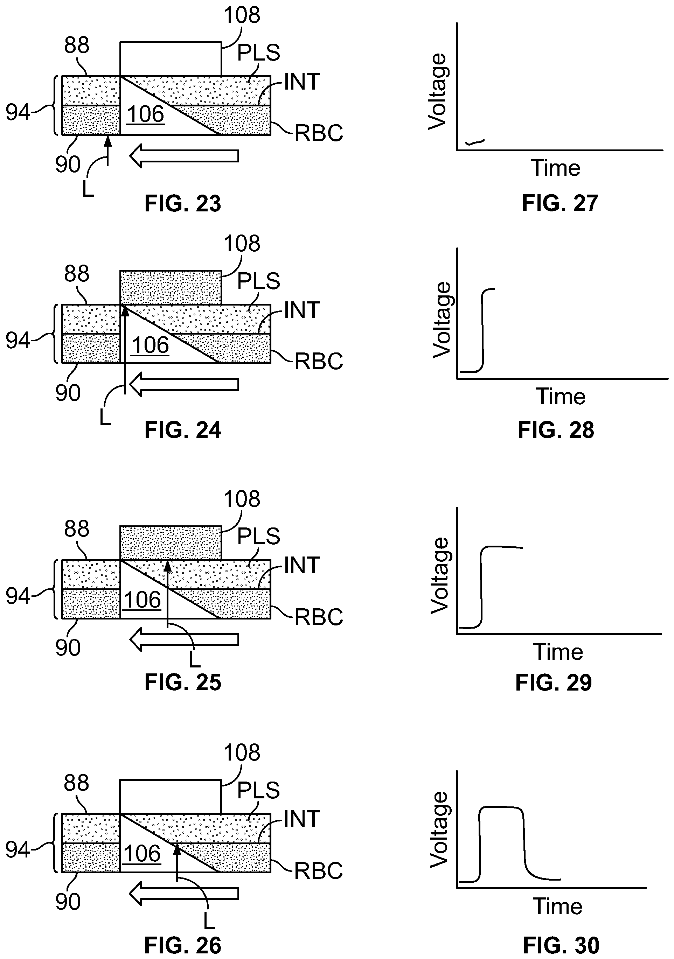

Figs. 23-26 show a portion of the ramp 106 being rotated into and through the initial path of the light L from the light source 28. Four specific events are shown: just before the ramp 106 is rotated into the path of the light L (Fig. 23), the ramp 106 first being rotated into the path of the light L (Fig. 24), just before the interface INT displayed on the ramp 106 is rotated into the path of the light L (Fig. 25), and just after the interface INT is rotated into the path of the light L (Fig. 26). Figs. 27-30 respectively illustrate the voltage output of the light detector 30 (corresponding to the signal that it transmits to the controller 18) during each of these events.

As described above, the light detector 30 will receive no light L from the light source 28 when the prismatic reflector 108 is out of alignment with the initial path of the light L from the light source 28, as shown in Fig. 23. Fig. 27 shows that the output voltage of the light detector 30 (i.e., the signal transmitted from the light detector 30 to the controller 18) at this time is in a low- or zero-state.

When the ramp 106 is first rotated into the path of light L from the light source 28 (Fig. 24), the light detector 30 may begin receiving light L. The amount of light L received by the light detector 30 depends upon the fluid on the ramp 106 encountered by the light L (i.e., the fluid in the channel 94 between the ramp 106 and the inner side wall portion 88 that the light L must traverse before being directed to the light detector 30). As described above, the less optically dense layer PLS occupies a certain percentage of the channel 94 adjacent to the inner side wall portion 88, while the optically dense layer RBC occupies a certain percentage of the channel 94 adjacent to the outer side wall portion 90 (with the interface INT positioned at the transition between the two separated blood component layers). The illustrated ramp 106 is closest to the inner side wall portion 88 at its left end (in the orientation of Figs. 23-26), while being farther spaced from the inner side wall portion 88 at its right end. At and adjacent to its left end, the ramp 106 will display only the fluid positioned closest to the inner side wall portion 88 (i.e., the less optically dense layer PLS), while the ramp 106 will display only the fluid positioned closest to the outer side wall portion 90 (i.e., the optically dense layer RBC) at and adjacent to its right end, as shown in Figs. 23-26.

At some point between its ends, the angled ramp 106 will be at a radial position where it will display the transition between the less optically dense layer PLS and the optically dense layer RBC (i.e., the interface INT). Hence, the location of the interface INT on the ramp 106 is dependent upon the percentage of the width of the ramp 106 that displays the less optically dense layer PLS (which is indicative of the percentage of the channel 94 occupied by the less optically dense layer PLS) and the percentage of the width of the ramp 106 that displays the optically dense layer RBC (which is indicative of the percentage of the channel 94 occupied by the optically dense layer RBC). It should be understood that the percentage of the ramp 106 occupied by the less optically dense layer PLS and by the optically dense layer RBC is not necessarily equal to the percentage of the channel 94 occupied by the less optically dense layer PLS and by the optically dense layer RBC, but that the percentage of the ramp 106 occupied by a separated blood component layer may be merely indicative of the percentage of the channel 94 occupied by that separated blood component layer.

In such an embodiment, as the ramp 106 is rotated into the path of the light L from the light source 28, the light L will first encounter the portion of the ramp 106 that is positioned closest to the inner side wall portion 88 (i.e., the section of the ramp 106 that most restricts the channel 94), as shown in Fig. 24. As described above, the less optically dense layer PLS will be positioned adjacent to the inner side wall portion 88 as it separates from the optically dense layer RBC, such that the fluid displayed on this radially innermost section of the ramp 106 (i.e., the fluid present in the channel 94 between the ramp 106 and the inner side wall portion 88) will be the less optically dense layer PLS. The light is substantially transmitted through the less optically dense layer PLS to the inner side wall portion 88, and through the light-transmissive inner side wall portion 88 to the prismatic reflector 108, which redirects the light L to the light detector 30. This causes the voltage output of the light detector 30 (i.e., the signal transmitted from the light detector 30 to the controller 18) to increase to a non-zero value or state, as shown in Fig. 28. Depending on the nature of the light L, the amount of light L received by the light detector 30 (and, hence, the magnitude of the voltage output) after the light L has passed through the less optically dense layer PLS may be greater than, less than, or equal to the amount of light L received by the light detector 30 after passing through saline during the calibration phase described above.

Further rotation of the ramp 106 through the path of light L from the light source 28 exposes the light L to portions of the ramp 106 that are increasingly spaced from the inner side wall portion 88 (i.e., the light L travels through portions of the channel 94 that are less restricted by the ramp 106 as the ramp 106 is rotated through the path of the light L). Up until the time that the interface INT on the ramp 106 is rotated into the path of the light L (as shown in Fig. 25), the only fluid in the channel 94 that the light L will have passed through will be the less optically dense layer PLS, such that a generally uniform level of light reaches the light detector 30 between the conditions shown in Figs. 24 and 25. Accordingly, the voltage output of the light detector 30 will be generally uniform (at an elevated level) the whole time that the ramp 106 passes through the path of the light L before being exposed to the interface INT, as shown in Fig. 29. The controller 18 may be programmed and/or configured to consider a signal that deviates from a maximum signal level (e.g., a 10% decrease) to be part of the elevated signal for purposes of calculating the pulse width of the signal. The controller 18 will treat a greater deviation (i.e., a greater decrease in the magnitude of the signal) as the end of the elevated signal for purposes of calculating the pulse width of the signal.

Just after the interface INT has been rotated into the path of light L from the light source 28, the light L will begin to encounter the optically dense layer RBC in the channel 94, as shown in Fig. 26). As described above, the optically dense layer RBC will be positioned adjacent to the outer side wall portion 90 as it separates from the less optically dense layer PLS, such that the optically dense layer RBC will not be displayed on the ramp 106 until the ramp 106 is spaced a greater distance away from the inner side wall portion 88 (i.e., toward the right end of the ramp 106 in the orientation of Figs. 23-26). Less light L is transmitted through the optically dense layer RBC than through the less optically dense layer PLS (which may include all or substantially all of the light L being absorbed by the optically dense layer RBC), such that the amount of light L that reaches the light detector 30 will decrease compared to the amount of light L that reaches the light detector 30 while traveling through only the less optically dense layer PLS in the channel 94 (Figs. 24 and 25).

When receiving less light L, the voltage output or signal from the light detector 30 will decrease to a lower level than when the light L was passing through only the less optically dense layer PLS in the channel 94, as shown in Fig. 30. When the light L encounters the optically dense layer RBC in the channel 94, the light detector 30 may be generating a signal or voltage output that is approximately equal to its zero-state (as in Fig. 27, when the light detector 30 is receiving no light L) or a signal or voltage output that is some degree less than the magnitude of the signal or voltage output generated while the light L encounters only the less optically dense layer PLS in the channel 94. The controller 18 may be programmed and/or configured to recognize this lower level signal as representing the presence of the optically dense layer RBC on the ramp 106 (and in the portion of the channel 94 being traversed by the light L) and treat this lower level signal as the end point of the elevated signal generated by the light detector 30 while light L passes through only the less optically dense layer PLS in the channel 94.

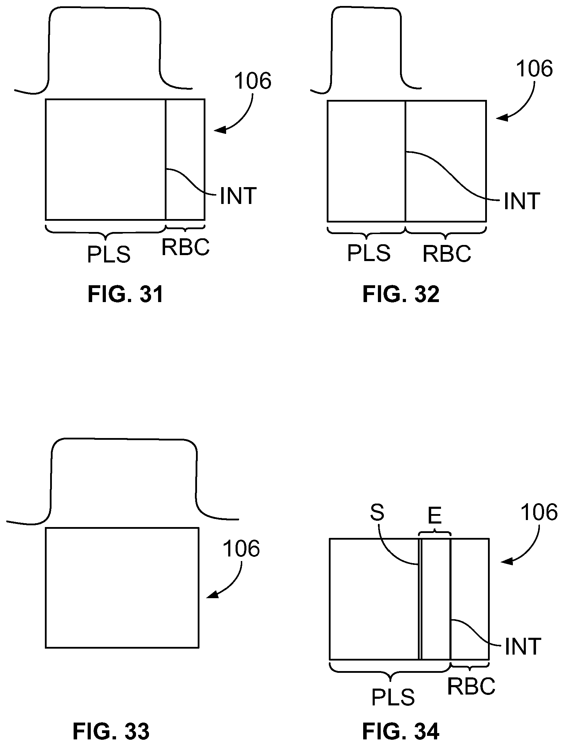

Thus, the pulse width of the elevated signal from the light detector 30 to the controller 18 (i.e., the time during which light L is traversing only the less optically dense layer PLS in the channel 94) is determined by the percentages of the ramp 106 that are occupied by the less optically dense layer PLS and the optically dense layer RBC. Accordingly, a greater pulse width of the signal from the light detector 30 to the controller 18 is associated with the less optically dense layer PLS occupying a larger portion of the ramp 106 (as shown in Fig. 31 from the point of view of the light source 28, which may correspond to the condition shown in Fig. 12) and will be indicative of a thinner optically dense layer RBC on the ramp 106 (and in the channel 94). Conversely, a signal from the light detector 30 to the controller 18 having a narrower pulse width is associated with the less optically dense layer PLS occupying a smaller portion of the ramp 106 (as shown in Fig. 32) and will be indicative of a thicker optically dense layer RBC on the ramp 106 (and in the channel 94).

The controller 18 may compare the pulse width of the signal to the pulse width generated during the calibration phase (described above and shown in Fig. 33), which corresponds to the pulse width when light L is transmitted to the light detector 30 over the entire width of the ramp 106. The pulse width of the signal generated by the light detector 30 during the calibration phase may be referred to as the saline calibration signal. Comparing these two pulse widths will indicate the percentage of the ramp 106 that is occupied by the less optically dense layer PLS and by the optically dense layer RBC, which information the controller 18 may use to determine the location of the interface INT within the channel 94. In particular, the interface position may be calculated as follows:

Interface position (%) = ((saline calibration pulse width - current plasma pulse widht) / saline calibration pulse widtr)*100          [Equation 1]

It will be seen that Equation 1 effectively calculates the percentage of the ramp 106 that is occupied by the optically dense layer RBC, as the difference between the two pulse widths corresponds to the length of time that the ramp 106 is rotated through the path of the light L without the light detector 30 received an elevated level of light L (i.e., the amount of time that the ramp 106 is rotated through the path of the light L while the optically dense layer RBC is present on the ramp 106).

3. Correction Of Interface Position

When the location of the interface INT on the ramp 106 has been determined, the interface control module compares the actual interface location with a desired interface location, which may be referred to as the setpoint S. The difference between the setpoint S and the calculated interface position may be referred to as the error signal E, which is shown in Fig. 34. It should be understood that so expressing the error signal E in terms of a targeted red blood cell percentage value (i.e., the percentage of the ramp 106 that is actually occupied by the optically dense layer RBC vs. the percentage of the ramp 106 which should be occupied by the optically dense layer RBC) is merely exemplary, and that the error signal E may be expressed or calculated in any of a number of other ways.

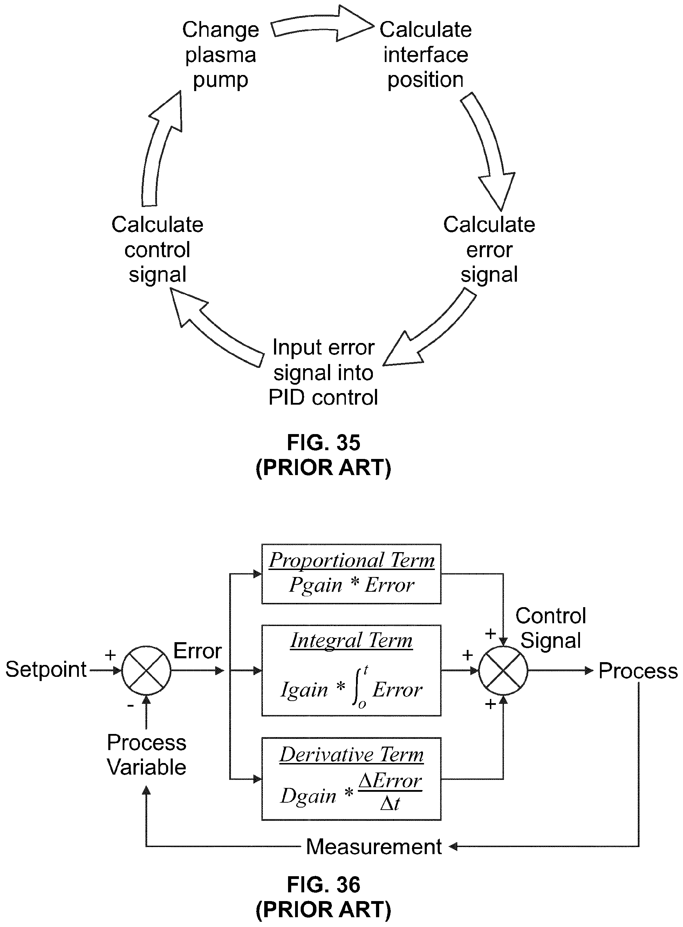

When the control value is expressed in terms of a targeted red blood cell percentage value, a negative error signal E indicates that the optically dense layer RBC on the ramp 106 is too large (as Fig. 11 shows). Conversely, a positive error signal indicates that the optically dense layer RBC on the ramp 106 is too small (as Figs. 12 and 34 show). In either case, the interface control module of the controller 18 inputs the error signal into a PID control algorithm to generate a control signal. The controller 18 uses the control signal to adjust an operational parameter accordingly, such as by adjusting the rate at which the plasma constituent is removed through the first outlet 102 under action of a pump of the blood separation device 10. All other things being equal, increasing the rate at which the plasma constituent is removed through the first outlet 102 will increase the thickness of the optically dense layer RBC, whereas decreasing the rate at which the plasma constituent is removed will decrease the thickness of the optically dense layer RBC. So adjusting the plasma outflow rate causes the interface INT to move toward the desired control position (as Fig. 10 shows), where the error signal is zero. Fig. 35 illustrates this conventional approach, with Fig. 36 illustrating a conventional PID control algorithm.

The Pgain, Igain, and Dgain values (collectively referred to as "the gains" or "PID gains") shown in Fig. 36 are the adjustable parameters of the PID control algorithm and are experimentally tuned to stabilize and maintain the position of the interface INT at the target position throughout a separation procedure. In conventional systems, the gains are set to constant predetermined values for all procedure and are never changed. However, as noted above, control instability may occur when blood parameters (particularly hematocrit and whole blood inflow rate) change. For example, when the hematocrit or inflow rate increase, the rate at which red blood cells enter the channel 94 increases and the thickness of the optically dense layer RBC will change (increase) more quickly. In this case, PID gains found to be appropriate and effective at lower hematocrit and inflow rate levels may no longer be appropriate and effective for moving the interface INT to the target position, thus resulting in instability.

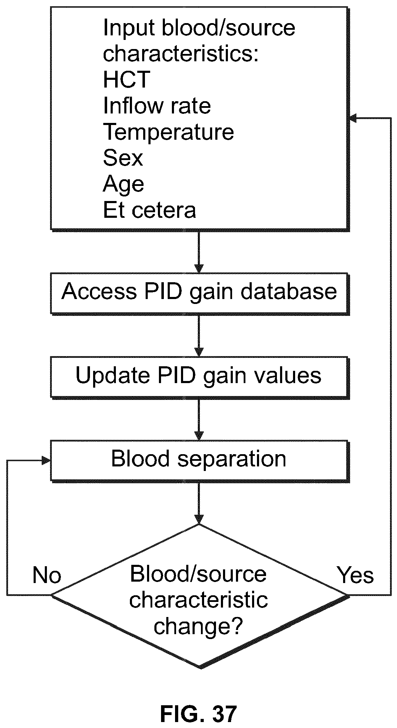

The approach according to the present disclosure modifies the conventional PID control algorithm of Fig. 36 by replacing the fixed gains with variable gains, which are adjusted based on the characteristics of the blood and/or blood source. The appropriate PID gains for different blood and blood source characteristics may be experimentally determined and stored in a database. At the beginning of a procedure, the controller 18 may access the database to select the appropriate PID gains based on various blood and/or blood source characteristics. Later, during blood separation, the controller 18 may again access the database to update the PID control algorithm with adjusted PID gains when one or more of the relevant blood and/or blood source characteristics have changed since the beginning of the procedure or since being last checked. This approach is illustrated in Fig. 37. When so modifying the PID control algorithm, it is possible for one, two, or all three of the individual gains to be changed, depending on the values stored in the database.

Fig. 37 shows exemplary blood characteristics (including hematocrit, inflow rate, and temperature) and blood source characteristics (including age and sex of a living blood source) that may be factors in determining the appropriate PID gains to be employed, but it should be understood that different and/or additional factors may be employed without departing from the scope of the present disclosure. While Fig. 37 illustrates characteristics of a living blood source, it should be understood that the principles described herein may be applied to non-living blood sources (e.g., blood packs or containers) as well. In general, factors or characteristics that affect the sedimentation of red blood cells and formation of the red blood cell bed will be of most interest, but any factor or characteristic experimentally found to be relevant to determination of the appropriate PID gains may be employed. These characteristics may be determined in any suitable manner, including by being provided to the controller 18 by an operator (e.g., by entering the age of a living blood source or the hematocrit of the blood at the beginning of a procedure) and being provided by a sensor of the blood separation device 10 (e.g., by periodically notifying the controller 18 of the hematocrit of the blood or the rate at which the blood is being pumped into the channel 94).

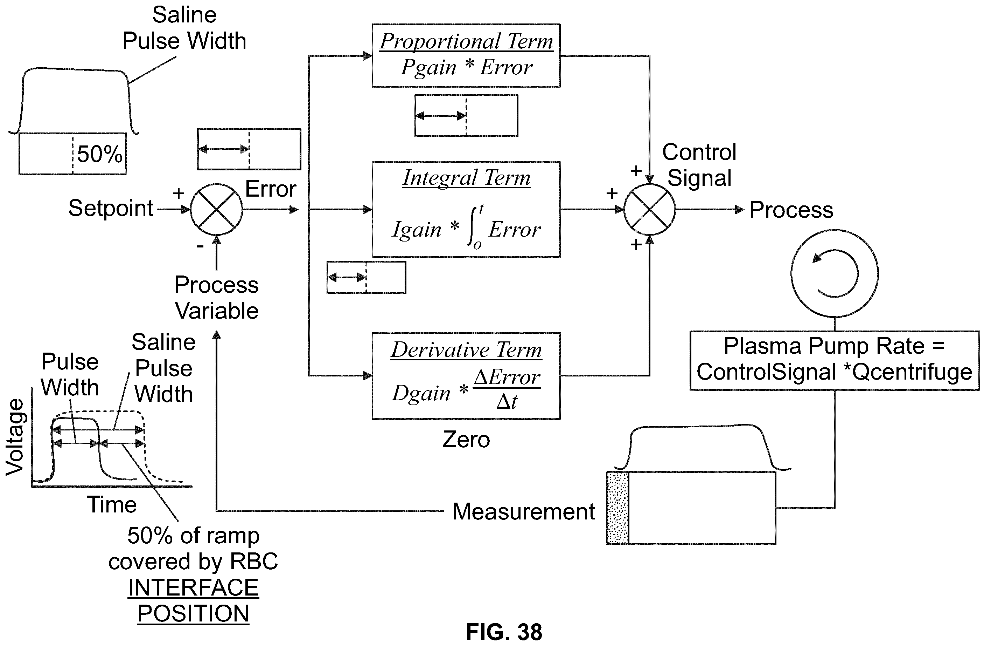

Figs. 38-41 illustrate an exemplary implementation of the approach shown in Fig. 37. Fig. 38 represents the beginning of a blood separation procedure (t=0), with the setpoint or target interface position corresponding to an equal thickness of plasma and red blood cells on the ramp 106. At this time, an interface position has not yet been established, so there is no error signal generated. In the illustrated embodiment, the control signal is a multiplier that is applied to the rate at which the plasma constituent is removed from the channel 94, in which case the control signal may be one. In other embodiments, the control signal may be a factor that is added to or subtracted from the rate at which the plasma constituent is removed from the channel 94, in which case the initial control signal may be zero. The controller 18 selects from the database appropriate PID gains based on blood and/or source characteristics, which are incorporated into the PID control algorithm at the beginning of blood separation. Alternatively, the controller 18 may employ default PID gains in the initial PID control algorithm.

Fig. 38 shows a condition in which the thickness of the red blood cell layer at the beginning of blood separation is too small (occupying 15-20% of the ramp 106, rather than 50%). As in the conventional approach (Figs. 35 and 36), the pulse width of the signal received by the light detector 30 is compared to the pulse width when the interface INT is at the target position in order to generate an error signal. The error signal is input into the PID control algorithm to generate a control signal, which is used to adjust the rate at which the plasma constituent is removed from the channel 94. In response to the illustrated condition, the rate would be increased in order to increase the thickness of the red blood cell layer. The magnitude of the change in outflow rate is based on values of the individual PID gains and the value of the error signal, with the proportional term being equal to the Pgain multiplied by the error signal (the current distance between the interface position and the target position), the integral term being equal to the Igain multiplied by the sum of all previous error signals, and the derivative term being equal to the Dgain multiplied by the rate at which the position of the interface INT has most recently changed (i.e., the difference between the two most recent error signals). Per an aspect of the present disclosure, the gains may be equal to the initial gain values (if none of the relevant blood and/or source characteristics have changed since the beginning of blood separation) or may be different from the initial values (if one or more of the relevant blood and/or source characteristics have changed).

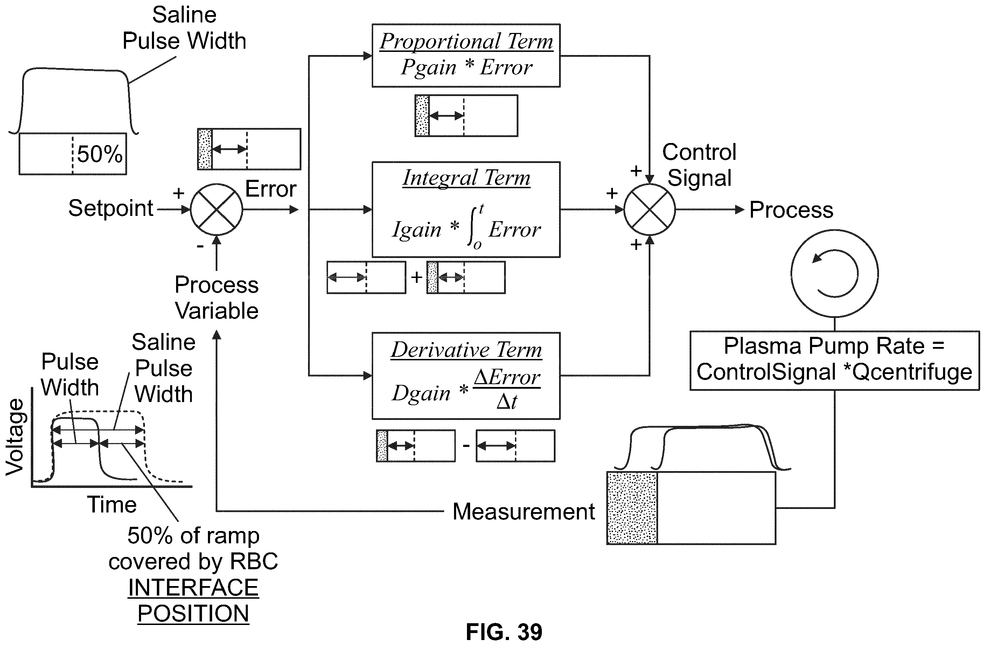

After the first control signal has been generated in response to the error signal and implemented, the interface position will move closer to the setpoint or target position, as shown in Fig. 39 (t=1). Fig. 39 shows that the thickness of the red blood cell layer remains undesirably low (occupying approximately 30% of the ramp 106), though the interface position is closer to the target position than at t=0 (in Fig. 38). A second error signal is generated and input into the PID control algorithm (with either the original or updated gains) to generate a second control signal, which is then used by the controller 18 to adjust the outflow rate of the plasma constituent. As described above, the controller 18 continues to assess whether any of the relevant blood and/or source characteristics have changed, with the database being consulted in response to any such changes so as to update one or more of the PID gains.

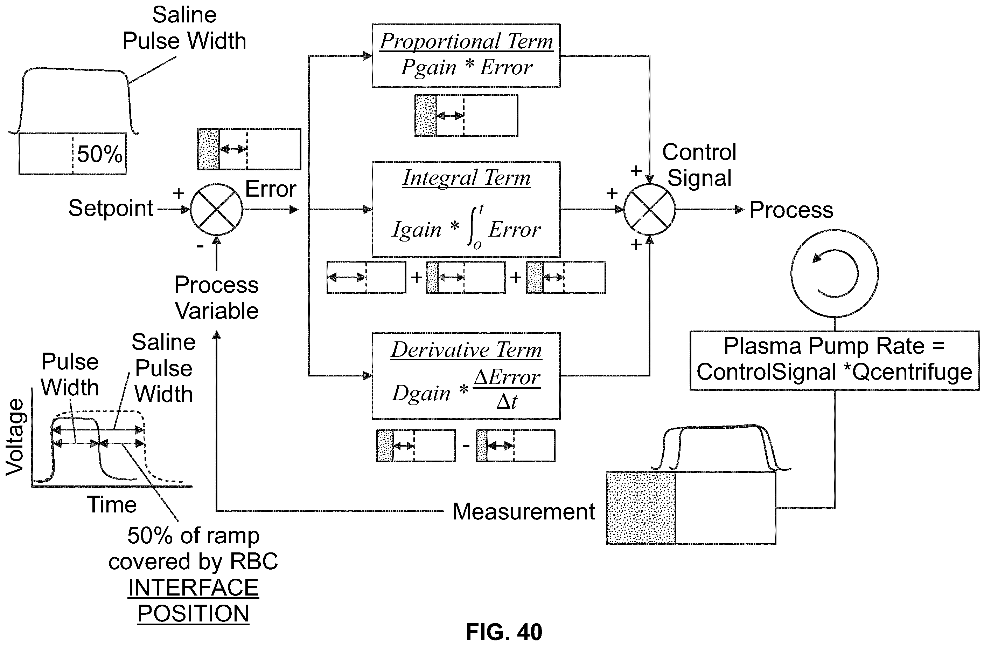

At t=2 (Fig. 40), the control loop is repeated, with the new (reduced) error signal being input into the PID control algorithm. The proportional term is updated in view of the new error signal, the integral term is updated to include the additional error signal (thus considering the error signals of Figs. 38 and 39), and the derivative term is updated in view of the magnitude of the interface position change brought about by implementation of the most recent control signal (as generated in Fig. 39). Once again, the controller 18 assesses whether any of the relevant blood and/or source characteristics have changed, with the database being consulted in response to any such changes so as to update one or more of the PID gains. As shown in Fig. 40, the thickness of the red blood cell layer remains undesirably low (occupying approximately 40% of the ramp 106), but the interface position is closer to the target position than at t=1 (Fig. 39).

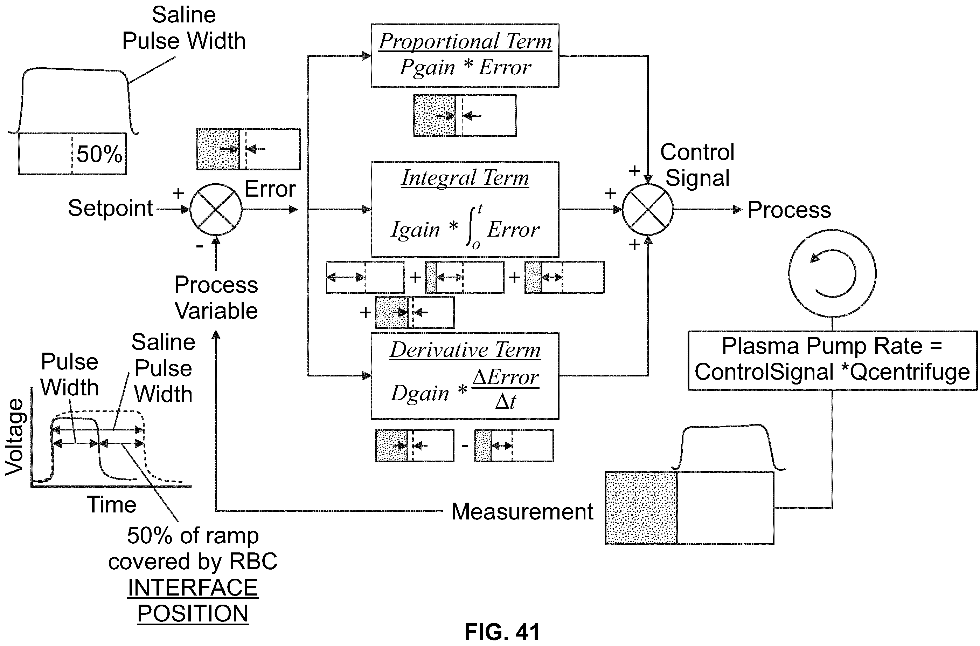

Finally, at t=3 (Fig. 41), the controller 18 again implements the control loop, with the new (twice reduced) error signal being input into the PID control algorithm. The proportional term is updated in view of the new error signal, the integral term is updated to include another error signal (thus considering the error signals of Figs. 38, 39, and 40), and the derivative term is updated in view of the magnitude of the interface position change brought about by implementation of the most recent control signal (as generated in Fig. 40). Before generating the control signal, the controller 18 again determines whether one or more of the PID gains should be updated in view of a change to any of the relevant blood and/or source characteristics. As shown in Fig. 41, the control signal generated by the PID control algorithm is effective at bringing the interface INT to the target position or setpoint, where the error signal is zero.

It is again emphasized that, unlike in the conventional approach of Fig. 35, the blood and source characteristics may be checked each time that the controller 18 implements the control loop, with the PID gains being updated when appropriate in view of changes to one or more blood or source characteristics, including the rate at which blood flows into the channel 94. By adjusting the PID gains during blood separation (rather than using the same unchanging gains, as is conventional), the controller 18 is able to more quickly and stably bring the interface INT to and retain the interface INT at the setpoint or target position.

It should be understood that routine illustrated in Figs. 38-41 is merely exemplary and that an interface control routine may vary without departing from the scope of the present disclosure. For example, rather than gradually moving the interface INT in one direction until it reaches the setpoint or target position, it may be the case that a control signal overcorrects the interface position, with the error signal changing from negative to positive or positive to negative. In this case, the control loop is simply repeated with the new error signal, which generates a new control signal that causes the interface INT to move in the opposite direction of its previous movement, thus moving it back toward and eventually to the setpoint or target position.

It should also be understood that the PID gains are not necessarily the only aspect of an interface adjustment routine that may be adjusted in view of a change to one or more blood or source characteristics. For example, the PID loop execution time may be adjusted (either instead of or in addition to an adjustment to one or more of the PID gains) in response to a change to one or more blood or source characteristics. The PID loop execution time may be increased or decreased in response to a change to one or more blood or source characteristics. In some circumstances, it may be advantageous to decrease the PID loop execution time when there has been a change in one or more blood or source characteristics in order to allow for a more rapid response to any future change. In other circumstances, it may be advantageous for a change in one or more blood or source characteristics to be addressed by an increase in the PID loop execution time, such as when the likelihood of a subsequent change is decreased by the occurrence of a given change in a blood or source characteristic.

The magnitude of a change in the PID loop execution time may vary without departing from the scope of the present disclosure and may be either dependent upon or independent of the nature and/or magnitude of the change in a blood or source characteristic. For example, a relatively large change in one or more blood or source characteristics may lead to a larger change in the PID loop execution time than a smaller change in the same blood or source characteristic(s). Alternatively, any change in one or more blood source characteristics (regardless of magnitude) may result in a change in the PID loop execution time having a fixed magnitude or duration.

## Claims

1. A blood separation device (10), comprising:

   a pump system (P1-P6);
   a centrifugal separator (16);
   an interface monitoring assembly; and
   a controller (18), wherein the controller (18) is configured to

      (a) actuate the pump system (P1-P6) to pump blood having a plurality of blood characteristics from a blood source having a plurality of source characteristics into the centrifugal separator (16) at an inflow rate,

(b) actuate the centrifugal separator (16) to separate the blood in the centrifugal separator (16) into two blood components, with an interface therebetween,

(c) actuate the pump system (P1-P6) to pump one of said blood components out of the centrifugal separator (16) at an outflow rate,

(d) actuate the interface monitoring assembly to determine a current position of the interface within the centrifugal separator (16),

(e) compare the current position of the interface to a target interface position and generate an error signal indicative of a distance between the current position of the interface and the target interface position,

(f) input the error signal into a proportional-integral-derivative control algorithm having a plurality of proportional-integral-derivative gains and an execution time to generate a control signal, and

(g) repeat (a) - (f) with said outflow rate being modified by the controller based at least in part on the control signal and with at least one of the proportional-integral-derivative gains or the execution time of said proportional-integral-derivative control algorithm being modified by the controller when a value of at least one of said plurality of blood characteristics, at least one of said plurality of source characteristics, and/or the inflow rate when executing (a) is different from the corresponding value when most recently executing (a).

2. The blood separation device (10) of claim 1, wherein one of said plurality of blood characteristics is a temperature of the blood.

3. The blood separation device (10) of any one of the preceding claims, wherein one of said plurality of blood characteristics is a hematocrit of the blood.

4. The blood separation device (10) of any one of the preceding claims, wherein

the controller (18) is programmed with or configured to access a database comprising a plurality of selectable proportional-integral-derivative gains to be incorporated into said proportional-integral-derivative control algorithm, and

each of said proportional-integral-derivative gains is based at least in part on at least one of said plurality of blood characteristics, at least one of said plurality of source characteristics, and/or the inflow rate.

5. The blood separation device (10) of claim 4, wherein the controller (18) is configured to, prior to beginning a blood separation procedure, select from the database the proportional-integral-derivative gains to be initially employed when beginning the blood separation procedure.

6. The blood separation device (10) of any one of the preceding claims, wherein the controller (18) is configured to modify the execution time of said proportional-integral-derivative control algorithm when said value of at least one of said plurality of blood characteristics, at least one of said plurality of source characteristics, and/or the inflow rate when executing (a) is different from the corresponding value when most recently executing (a).

7. The blood separation device (10) of any one of the preceding claims, wherein the controller (18) is configured to modify at least one of the proportional-integral-derivative gains and the execution time of said proportional-integral-derivative control algorithm when said value of at least one of said plurality of blood characteristics, at least one of said plurality of source characteristics, and/or the inflow rate when executing (a) is different from the corresponding value when most recently executing (a).

8. The blood separation device (10) of any one of the preceding claims, wherein

the blood source is a living blood source, and
one of said plurality of source characteristics is the age and/or the sex of the blood source.

9. The blood separation device (10) of any one of claims 1-7, wherein the blood source is a non-living blood source.

10. A method for separating blood, comprising:

(a) conveying blood having a plurality of blood characteristics from a non-living blood source having a plurality of source characteristics into a centrifugal separator (16) at an inflow rate;

(b) separating the blood in the centrifugal separator (16) into two blood components, with an interface therebetween;

(c) conveying one of said blood components out of the centrifugal separator (16) at an outflow rate;

(d) determining a current position of the interface within the centrifugal separator (16);

(e) comparing the current position of the interface to a target interface position and generating an error signal indicative of a distance between the current position of the interface and the target interface position;

(f) inputting the error signal into a proportional-integral-derivative control algorithm having a plurality of proportional-integral-derivative gains and an execution time to generate a control signal; and

(g) repeating (a) - (f) with said outflow rate being modified based at least in part on the control signal and with at least one of the proportional-integral-derivative gains or the execution time of said proportional-integral-derivative control algorithm being modified when a value of at least one of said plurality of blood characteristics, at least one of said plurality of source characteristics, and/or the inflow rate when executing (a) is different from the corresponding value when most recently executing (a).

11. The method of claim 10, wherein one of said plurality of blood characteristics is a temperature and/or a hematocrit of the blood.

12. The method of any one of claims 10-11, wherein

modifying the proportional-integral-derivative control algorithm includes selecting from a database at least one of a plurality of selectable proportional-integral-derivative gains to be incorporated into said proportional-integral-derivative control algorithm, and

each of said proportional-integral-derivative gains is based at least in part on at least one of said plurality of blood characteristics, at least one of said plurality of source characteristics, and/or the inflow rate.

13. The method of claim 12, further comprising, prior to beginning blood separation, selecting from the database the proportional-integral-derivative gains to be initially employed when beginning blood separation.

14. The method of any one of claims 10-13, wherein

the execution time of said proportional-integral-derivative control algorithm is modified when said value of at least one of said plurality of blood characteristics, at least one of said plurality of source characteristics, and/or the inflow rate when executing (a) is different from the corresponding value when most recently executing (a).

15. The method of any one of claims 10-14, wherein

at least one of the proportional-integral-derivative gains and the execution time of said proportional-integral-derivative control algorithm are modified when said value of at least one of said plurality of blood characteristics, at least one of said plurality of source characteristics, and/or the inflow rate when executing (a) is different from the corresponding value when most recently executing (a).

**Patentansprüche**

1. Blutseparationsvorrichtung (10), umfassend:

ein Pumpsystem (P1-P6);
einen Zentrifugalseparator (16);
eine Grenzflächen-Überwachungseinheit; und
einen Controller (18),
wobei der Controller (18) dazu konfiguriert ist,

(a) das Pumpsystem (P1-P6) anzusteuern, um Blut mit einer Vielzahl von Blutmerkmalen aus einer Blutquelle mit einer Vielzahl von Quellenmerkmalen mit einer Zuflussrate in den Zentrifugalseparator (16) zu fördern,

(b) den Zentrifugalseparator (16) anzusteuern, um das Blut im Zentrifugalseparator (16) in zwei Blutkomponenten mit einer dazwischenliegenden Grenzfläche zu trennen,

(c) das Pumpsystem (P1-P6) anzusteuern, um eine der Blutkomponenten mit einer Abflussrate aus dem Zentrifugalseparator (16) zu fördern,

(d) die Grenzflächen-Überwachungseinheit anzusteuern, um eine aktuelle Position der Grenzfläche innerhalb des Zentrifugalseparators (16) zu bestimmen,

(e) die aktuelle Position der Grenzfläche mit einer Soll-Grenzflächenposition zu vergleichen und ein Fehlersignal zu erzeugen, das einen Abstand zwischen der aktuellen Position der Grenzfläche und der Soll-Grenzflächenposition anzeigt,

(f) das Fehlersignal in einen Proportional-Integral-Differential-Regelalgorithmus mit einer Vielzahl von Proportional-Integral-Differential-Regelverstärkungen und einer Ausführungszeit einzuspeisen, um ein Steuersignal zu erzeugen, und

(g) die Schritte (a)-(f) zu wiederholen, wobei die Abflussrate vom Controller zumindest teilweise auf Grundlage des Steuersignals verändert wird und wobei mindestens eine der Proportional-Integral-Differential-Regelverstärkungen oder die Ausführungszeit des Proportional-Integral-Differential-Regelalgorithmus vom Controller verändert wird, wenn ein Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

2. Blutseparationsvorrichtung (10) nach Anspruch 1, wobei eines der Vielzahl an Blutmerkmalen eine Temperatur des Blutes ist.

3. Blutseparationsvorrichtung (10) nach einem der vorhergehenden Ansprüche, wobei eines der Vielzahl an Blutmerkmalen ein Hämatokrit des Blutes ist.

4. Blutseparationsvorrichtung (10) nach einem der vorhergehenden Ansprüche,

wobei der Controller (18) mit einer Datenbank programmiert oder konfiguriert ist, auf eine solche zuzugreifen, wobei die Datenbank eine Vielzahl auswählbarer Proportional-Integral-Differential-Regelverstärkungen umfasst, die in den Proportional-Integral-Differential-Regelalgorithmus zu integrieren sind, und

wobei jede der Proportional-Integral-Differential-Regelverstärkungen zumindest teilweise auf mindestens einem der Vielzahl an Blutmerkmalen, mindestens einem der Vielzahl an Quellenmerkmalen und/oder der Zuflussrate basiert.

5. Blutseparationsvorrichtung (10) nach Anspruch 4, wobei der Controller (18) dazu konfiguriert ist, vor Beginn eines Blutseparationsverfahrens aus der Datenbank die Proportional-Integral-Differential-Regelverstärkungen auszuwählen, die zu Beginn des Blutseparationsverfahrens zunächst verwendet werden.

6. Blutseparationsvorrichtung (10) nach einem der vorhergehenden Ansprüche, wobei der Controller (18) dazu konfiguriert ist, die Ausführungszeit des Proportional-Integral-Differential-Regelalgorithmus zu verändern, wenn der Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

7. Blutseparationsvorrichtung (10) nach einem der vorhergehenden Ansprüche, wobei der Controller (18) dazu konfiguriert ist, mindestens eine der Proportional-Integral-Differential-Regelverstärkungen und die Ausführungszeit des Proportional-Integral-Differential-Regelalgorithmus zu verändern, wenn der Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

8. Blutseparationsvorrichtung (10) nach einem der vorhergehenden Ansprüche, wobei

die Blutquelle eine lebende Blutquelle ist, und
eines der Vielzahl an Quellenmerkmalen das Alter und/oder das Geschlecht der Blutquelle ist.

9. Blutseparationsvorrichtung (10) nach einem der Ansprüche 1-7, wobei die Blutquelle eine nicht-lebende Blutquelle ist.

10. Verfahren zur Blutseparation, umfassend:

(a) Fördern von Blut mit einer Vielzahl von Blutmerkmalen aus einer nichtlebenden Blutquelle mit einer Vielzahl von Quellenmerkmalen mit einer Zuflussrate in einen Zentrifugalseparator (16);

(b) Trennen des Blutes im Zentrifugalseparator (16) in zwei Blutkomponenten mit einer dazwischenliegenden Grenzfläche;

(c) Fördern einer der Blutkomponenten mit einer Abflussrate aus dem Zentrifugalseparator (16);

(d) Bestimmen einer aktuellen Position der Grenzfläche innerhalb des Zentrifugalseparators (16);

(e) Vergleichen der aktuellen Position der Grenzfläche mit einer Soll-Grenzflächenposition und Erzeugen eines Fehlersignals, das einen Abstand zwischen der aktuellen Position der Grenzfläche und der Soll-Grenzflächenposition anzeigt;

(f) Einspeisen des Fehlersignals in einen Proportional-Integral-Differential-Regelalgorithmus mit einer Vielzahl von Proportional-Integral-Differential-Regelverstärkungen und einer Ausführungszeit zur Erzeugung eines Steuersignals; und

(g) Wiederholen der Schritte (a)-(f), wobei die Abflussrate zumindest teilweise auf Grundlage des Steuersignals verändert wird und wobei mindestens eine der Proportional-Integral-Differential-Regelverstärkungen oder die Ausführungszeit des Proportional-Integral-Differential-Regelalgorithmus verändert wird, wenn ein Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

11. Verfahren nach Anspruch 10, wobei eines der Vielzahl an Blutmerkmalen eine Temperatur und/oder ein Hämatokrit des Blutes ist.

12. Verfahren nach einem der Ansprüche 10-11, wobei

das Modifizieren des Proportional-Integral-Differential-Regelalgorithmus das Auswählen mindestens einer aus einer Vielzahl auswählbarer Proportional-Integral-Differential-Regelverstärkungen aus einer Datenbank zur Integration in den Proportional-Integral-Differential-Regelalgorithmus umfasst, und

jede der Proportional-Integral-Differential-Regelverstärkungen zumindest teilweise auf mindestens einem der Vielzahl an Blutmerkmalen, mindestens einem der Vielzahl an Quellenmerkmalen und/oder der Zuflussrate basiert.

13. Verfahren nach Anspruch 12, ferner umfassend, vor Beginn der Blutseparation aus der Datenbank die Proportional-Integral-Differential-Regelverstärkungen auszuwählen, die zu Beginn der Blutseparation zunächst verwendet werden.

14. Verfahren nach einem der Ansprüche 10-13, wobei die Ausführungszeit des Proportional-Integral-Differential-Regelalgorithmus verändert wird, wenn der Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

15. Verfahren nach einem der Ansprüche 10-14, wobei mindestens eine der Proportional-Integral-Differential-Regelverstärkungen und die Ausführungszeit des Proportional-Integral-Differential-Regelalgorithmus verändert werden, wenn der Wert von mindestens einem der Vielzahl an Blutmerkmalen, mindestens einem der Vielzahl an Quellenmerkmalen und/oder der Zuflussrate bei der Ausführung von Schritt (a) von dem entsprechenden Wert bei der zuletzt ausgeführten Durchführung von Schritt (a) abweicht.

**Revendications**

1. Dispositif de séparation de sang (10), comprenant :

un système de pompes (P1-P6) ;
un séparateur centrifuge (16) ;
un ensemble de surveillance d'interface ; et
un contrôleur (18),
dans lequel le contrôleur (18) est configuré pour :

(a) actionner le système de pompes (P1-P6) afin de pomper du sang présentant une pluralité de caractéristiques de sang à partir d'une source de sang présentant une pluralité de caractéristiques de source vers le séparateur centrifuge (16) à un débit d'entrée,

(b) actionner le séparateur centrifuge (16) afin de séparer le sang dans le séparateur centrifuge (16) en deux composants sanguins, avec une interface de séparation entre ceux-ci,

(c) actionner le système de pompes (P1-P6) afin de pomper un desdits composants sanguins hors du séparateur centrifuge (16) à un débit de sortie,

(d) actionner l'ensemble de surveillance d'interface afin de déterminer une position actuelle de l'interface à l'intérieur du séparateur centrifuge (16),

(e) comparer la position actuelle de l'interface à une position cible de l'interface et générer un signal d'erreur indiquant une distance entre la position actuelle de l'interface et la position cible de l'interface de séparation,

(f) introduire le signal d'erreur dans un algorithme de commande proportionnelle-intégrale-dérivée comprenant une pluralité de gains proportionnels-intégraux-dérivés et un temps d'exécution afin de générer un signal de commande, et

(g) répéter les étapes (a) à (f), ledit débit de sortie étant modifié par le contrôleur sur la base au moins en partie du signal de commande et au moins un des gains proportionnels-intégraux-dérivés ou le temps d'exécution dudit algorithme de commande proportionnelle-intégrale-dérivée étant modifié par le contrôleur lorsqu'une valeur d'au moins une desdites caractéristiques de sang, d'au moins une desdites caractéristiques de la source et/ou du débit d'entrée lors de l'exécution de l'étape (a) est différente de la valeur correspondante lors de la dernière exécution de l'étape (a).

2. Dispositif de séparation de sang (10) selon la revendication 1, dans lequel une desdites caractéristiques de sang est une température du sang.

3. Dispositif de séparation de sang (10) selon l'une quelconque des revendications précédentes, dans lequel une desdites caractéristiques de sang est un hématocrite du sang.

4. Dispositif de séparation de sang (10) selon l'une quelconque des revendications précédentes, dans lequel

le contrôleur (18) est programmé avec ou configuré pour accéder à une base de données comprenant une pluralité de gains proportionnels-intégraux-dérivés sélectionnables à incorporer dans ledit algorithme de commande proportionnelle-intégrale-dérivée, et

chacun desdits gains proportionnels-intégraux-dérivés est basé au moins en partie sur au moins une desdites caractéristiques de sang, au moins une desdites caractéristiques de source et/ou le débit d'entrée.

5. Dispositif de séparation de sang (10) selon la revendication 4, dans lequel le contrôleur (18) est configuré pour, avant le début d'une procédure de séparation de sang, sélectionner à partir de la base de données les gains proportionnels-intégraux-dérivés à être utilisés initialement au début de la procédure de séparation de sang.

6. Dispositif de séparation de sang (10) selon l'une quelconque des revendications précédentes, dans lequel le contrôleur (18) est configuré pour modifier le temps d'exécution dudit algorithme de commande proportionnelle-intégrale-dérivée lorsque ladite valeur d'au moins une desdites caractéristiques de sang, d'au moins une desdites caractéristiques de source et/ou du débit d'entrée lors de l'exécution de l'étape (a) est différente de la valeur correspondante lors de la dernière exécution de l'étape (a).

7. Dispositif de séparation de sang (10) selon l'une quelconque des revendications précédentes, dans lequel le contrôleur (18) est configuré pour modifier au moins un des gains proportionnels-intégraux-dérivés et le temps d'exécution dudit algorithme de commande proportionnelle-intégrale-dérivée lorsque ladite valeur d'au moins une desdites caractéristiques de sang, d'au moins une desdites caractéristiques de source et/ou du débit d'entrée lors de l'exécution de l'étape (a) est différente de la valeur correspondante lors de la dernière exécution de l'étape (a).

8. Dispositif de séparation de sang (10) selon l'une quelconque des revendications précédentes, dans lequel la source de sang est une source de sang vivante, et une desdites caractéristiques de la source est l'âge et/ou le sexe de la source de sang.

9. Dispositif de séparation de sang (10) selon l'une quelconque des revendications 1 à 7, dans lequel la source de sang est une source de sang non vivante.

10. Procédé de séparation de sang, comprenant :

(a) l'acheminement de sang présentant une pluralité de caractéristiques de sang à partir d'une source de sang non vivante présentant une pluralité de caractéristiques de source vers un séparateur centrifuge (16) à un débit d'entrée ;

(b) la séparation du sang dans le séparateur centrifuge (16) en deux composants sanguins, avec une interface de séparation entre ceux-ci ;

(c) l'acheminement d'un desdits composants sanguins hors du séparateur centrifuge (16) à un débit de sortie ;

(d) la détermination d'une position actuelle de l'interface à l'intérieur du séparateur centrifuge (16) ;

(e) la comparaison de la position actuelle de l'interface à une position cible d'interface et la génération d'un signal d'erreur indiquant une distance entre la position actuelle de l'interface et la position cible d'interface;

(f) l'introduction du signal d'erreur dans un algorithme de commande proportionnelle-intégrale-dérivée comprenant une pluralité de gains proportionnels-intégraux-dérivés et un temps d'exécution afin de générer un signal de commande ; et

(g) la répétition des étapes (a) à (f), ledit débit de sortie étant modifié sur la base au moins en partie du signal de commande et au moins un des gains proportionnels-intégraux-dérivés ou le temps d'exécution dudit algorithme de commande proportionnelle-intégrale-dérivée étant modifié lorsque la valeur d'au moins une desdites caractéristiques de sang, d'au moins une desdites caractéristiques de source et/ou du débit d'entrée lors de l'exécution de l'étape (a) est différente de la valeur correspondante lors de la dernière exécution de l'étape (a).

11. Procédé selon la revendication 10, dans lequel l'une desdites caractéristiques du sang est une température et/ou un hématocrite du sang.

12. Procédé selon l'une quelconque des revendications 10 à 11, dans lequel

la modification de l'algorithme de commande proportionnelle-intégrale-dérivée comprend la sélection, à partir d'une base de données, d'au moins un d'une pluralité de gains proportionnels-intégraux-dérivés sélectionnables à incorporer dans ledit algorithme de commande proportionnelle-intégrale-dérivée, et

dans lequel chacun desdits gains proportionnels-intégraux-dérivés est basé au moins en partie sur au moins une desdites caractéristiques de sang, au moins une desdites caractéristiques de source et/ou le débit d'entrée.

13. Procédé selon la revendication 12, comprenant en outre, avant le début de la séparation du sang, la sélection à partir de la base de données des gains proportionnels-intégraux-dérivés à être utilisés initialement au début de la séparation du sang.

14. Procédé selon l'une quelconque des revendications 10 à 13, dans lequel le temps d'exécution dudit algorithme de commande proportionnelle-intégrale-dérivée est modifié lorsque ladite valeur d'au moins une desdites caractéristiques de sang, d'au moins une desdites caractéristiques de source et/ou du débit d'entrée lors de l'exécution de l'étape (a) est différente de la valeur correspondante lors de la dernière exécution de l'étape (a).

15. Procédé selon l'une quelconque des revendications 10 à 14, dans lequel au moins un des gains proportionnels-intégraux-dérivés et le temps d'exécution dudit algorithme de commande proportionnelle-intégrale-dérivée sont modifiés lorsque ladite valeur d'au moins une desdites caractéristiques de sang, d'au moins une desdites caractéristiques de source et/ou du débit d'entrée lors de l'exécution de l'étape (a) est différente de la valeur correspondante lors de la dernière exécution de l'étape (a).

**FIG. 1**

FIG. 2

**FIG. 3**

FIG. 4

EP 4 306 144 B1

FIG. 6

FIG. 5

**FIG. 7**

**FIG. 13**

**FIG. 14**

**FIG. 8**

FIG. 9

**FIG. 10**

**FIG. 11**

**FIG. 12**

FIG. 15

FIG. 16

FIG. 17

FIG. 18

FIG. 19

FIG. 20

FIG. 21

FIG. 22

**FIG. 23**

**FIG. 27**

**FIG. 24**

**FIG. 28**

**FIG. 25**

**FIG. 29**

**FIG. 26**

**FIG. 30**

106

INT

PLS RBC

**FIG. 31**

106

INT

PLS RBC

**FIG. 32**

106

**FIG. 33**

S E 106

INT

PLS RBC

**FIG. 34**

**FIG. 35**
**(PRIOR ART)**

**FIG. 36**
**(PRIOR ART)**

Input blood/source
characteristics:
HCT
Inflow rate
Temperature
Sex
Age
Et cetera

Access PID gain database

Update PID gain values

Blood separation

No ⟨ Blood/source characteristic change? ⟩ Yes

**FIG. 37**

FIG. 38

FIG. 39

FIG. 40

FIG. 41

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190201916 A
- EP 3015126 A1
- WO 2021194824 A1
- US 8075468 B
- US 5868696 A
- US 6419822 B
- US 5632893 A